# EUROPEAN PATENT SPECIFICATION

(11) **EP 4 525 889 B1**
(45) Date of publication and mention of the grant of the patent: **22.07.2026**
(21) Application number: 23728809.7
(22) Date of filing: 02.06.2023
(51) Int. Cl.: A61K 33/14, A61K 33/00, A61K 33/06, A61P 29/00, A61P 1/00, A61P 3/02, A61P 3/04, A61P 9/10, A61P 19/02, A61P 21/00, A61P 25/02, A61P 25/06, A61P 25/16, A61P 25/28, A61P 31/14, A61P 37/00, A61P 17/02

(54) **MULTI-IONIC COMPLEX FOR THE PREVENTION OR TREATMENT OF INFLAMMATION**
MULTI-IONISCHER KOMPLEX ZUR VORBEUGUNG ODER BEHANDLUNG VON ENTZÜNDUNGEN
COMPLEXE MULTI-IONIQUE POUR LA PRÉVENTION OU LE TRAITEMENT DE L'INFLAMMATION

(30) Priority: 03.06.2022 BE 202205436
(43) Date of publication of application: 26.03.2025
(73) Proprietor: YNIOS Pharma, 1310 La Hulpe (BE)
(72) Inventor: VAN WESEPOEL, Philippe, 4500 Huy (BE); PLUCKER, Jean-François, 1180 Uccle (BE)
(74) Representative: Kirkpatrick
(86) International application number: PCT/EP2023/064818
(87) International publication number: WO 2023/232997

(56) References cited:
- WO-A1-2013/081366
- WO-A1-2022/254042
- CN-A- 108 310 287
- FR-A1- 3 113 584
- KR-A- 20200 109 179
- MADIREDDY SAHITHI ET AL: "Therapeutic Interventions to Mitigate Mitochondrial Dysfunction and Oxidative Stress-Induced Damage in Patients with Bipolar Disorder", INTERNATIONAL JOURNAL OF MOLECULAR SCIENCES, vol. 23, no. 3, 6 February 2022 (2022-02-06), pages 1844, XP093011949, DOI: 10.3390/ijms23031844
- SPUCH CARLOS ET AL: "Does Lithium Deserve a Place in the Treatment Against COVID-19? A Preliminary Observational Study in Six Patients, Case Report", FRONTIERS IN PHARMACOLOGY, vol. 11, 27 August 2020 (2020-08-27), XP055933102, DOI: 10.3389/fphar.2020.557629
- VERONESE NICOLA ET AL: "Effect of Magnesium Supplementation on Inflammatory Parameters: A Meta-Analysis of Randomized Controlled Trials", NUTRIENTS, vol. 14, no. 3, 5 February 2022 (2022-02-05), pages 679, XP093011950, DOI: 10.3390/nu14030679
- RASTMANESH R ET AL: "A Pilot Study of Potassium Supplementation in the Treatment of Hypokalemic Patients With Rheumatoid Arthritis: A Randomized, Double-Blinded, Placebo-Controlled Trial", JOURNAL OF PAIN, SAUNDERS, PHILADELPHIA, PA, US, vol. 9, no. 8, 1 August 2008 (2008-08-01), pages 722 - 731, XP023171721, ISSN: 1526-5900, [retrieved on 20080512], DOI: 10.1016/J.JPAIN.2008.03.006
- KHALILI HAMED ET AL: "Identification and Characterization of a Novel Association between Dietary Potassium and Risk of Crohn's Disease and Ulcerative Colitis", FRONTIERS IN IMMUNOLOGY, vol. 7, 7 December 2016 (2016-12-07), XP093012089, DOI: 10.3389/fimmu.2016.00554

## Description

### Field of invention

The invention falls within the field of a therapeutic response to prevent and / or treat inflammation and restore cellular homeostasis.

### Context

When a cell suffers damage or is subjected to stress (of psychic, metabolic, chemical origin...), a depletion of intracellular magnesium Mg²⁺ follows, with concentrations that can drop by 40 to 60% in the case of damage (Neuropsychiatric Disease and Treatment 2017: 13 275-302*).* In fact, small fluctuations in the intracellular concentration of Mg²⁺ can impact cellular processes. Dysregulation of Mg²⁺ is frequently observed in patients suffering from diabetes, neurodegenerative diseases as well as metabolic syndrome... *(*Magnesium is a key player in neuronal maturation and neuropathology, Int. J. Mol. Sci. 2019, 20, 3439*).*

Magnesium acts as a cofactor in more than 300 enzymatic reactions where it is crucial for the metabolism of adenosine triphosphate (ATP), a source of energy *(*Magnesium in Prevention and Therapy, Nutrients 2015, 7*).*

Magnesium has powerful anti-oxidant, anti-necrotic and anti-apoptotic effects. Mg²⁺ is itself largely cytoprotective, cardioprotective, and neuroprotective against a wide range of insults (Neuropsychiatric Disease and Treatment, 2017:13 275-302).

Magnesium, the fourth most common mineral in the body, also plays an essential role in nerve transmission and neuromuscular conduction. Low magnesium levels are associated with elevated glutaminergic neurotransmission, leading to a favorable environment for excitotoxicity, which can lead to oxidative stress and neuronal cell death. This process is involved in several neurological disorders, such as chronic pain *(*The Role of Magnesium in Neurological Disorders, Nutrients, 2018, 10,730*).*

A magnesium deficiency negatively impacts the insulin resistance index (HOMA-IR). Magnesium is also essential for protein synthesis, including DNA and RNA synthesis (Magnesium in Prevention and Therapy, Nutrients, 2015, 7). It regulates the transmembrane flows of potassium and calcium.

Based on experiments in which animals are deprived of lithium, lithium is considered an essential nutrient for the functioning of the human body. Unlike other biologically active ions, the concentration of lithium in fluids of multicellular animals is not tightly regulated. Its concentration can vary widely.

Lithium, while having a strong biological activity, is tolerated at highly variable concentrations of body fluid. The lack of biological regulation of lithium appears to be due to the absence of lithium-specific binding sites and selectivity filters. Rather, lithium exerts its myriad of physiological and biochemical effects by competing with other elements for relatively specific macromolecular sites of other cations, most notably sodium and magnesium (Towards a unified understanding of Lithium action, Topical Review, 2017, 586).

Lithium and magnesium possess similar ionic rays (0.60 and 0.65 Angström, respectively) and similar physicochemical properties allowing them to compete successfully to bind to several magnesium-dependent enzyme sites (Lithium: the pharmacodynamic actions of the amazing ion Ther. Adv. Psychopharmacol. (2013) 3(3) 163-176).

These ionic rays allow lithium and magnesium to easily cross cell membranes.

The Li⁺ ion activates survival and recovery mechanisms such as inositol monophosphatase (IMPase)/inositol polyphosphate 1-phosphatase (IPPase), glycogen synthase kinase 3 (GSK-3) inhibition. Lithium thus produces remarkable protective, anti-apoptotic, anti-anoxic, cellular plasticity and resilience responses. (A fully integrated new paradigm for lithium's mode of action - lithium utilizes latent cellular fail-safe mechanisms, Neuropsychiatric Disease and Treatment 2017:13 275-302).

The lithium glycogen synthase kinase 3 (GSK-3β) target is a serine/threonine kinase, which plays a role in regulating cellular metabolism in mammals. It regulates neurogenesis, neuronal polarization and axon growth in the developing central nervous system. It is constitutively active in all tissues (Role of glycogen synthase kinase-3b in ketamine-induced developmental neuroapoptosis in rats, British Journal of Anaesthesia 110 (S1): i3-i9 (2013*)).*

GSK3β is also an activator of the nucleotide-binding oligomerization domain-like receptor family pyrin domain-containing-3 (NLRP3) inflammasome (S Arumugam et al Cell Death Differ 2022). NLRP3 is an intracellular sensor for microbial motifs and endogenous and environmental danger signals inducing the NLRP3 inflammasome assembly and activation. NLRP3 assembly and activation occurs in 2 steps. First the priming signal (pathogen-associated molecular patterns (PAMPs) or damage-associated molecular patterns (DAMPs)) mediated by Toll-Like Receptor (TLR) or Tumor Necrosis Factor Receptor (TNFR) and leading to upregulation of NLRP3 and pro-IL-1B levels through activation of the transcription factor nuclear factor kappa-light-chain-enhancer (NF-κB) involved in innate immunity. The second step signal (PAMPs, DAMPs, extracellular ATP-P2X7R, ROS, intracellular Calcium levels, etc.) leads to NLRP3 activation and assembly. The NLRP3 inflammasome promotes caspase-1-dependent release of pro-inflammatory cytokines IL-1β and IL-18 as well as cell gasdermin-D-mediated cell death by pyroptosis (KV Swanson et al Nat Rev Immunol 2019*).* NLRP3 inflammasome is involved in many acute and chronic inflammatory diseases such as myocardial infarction, colitis, diabetes, steatohepatitis, Alzheimer's disease, traumatic brain injury, atherosclerosis, stroke, cancer and many others (Y Li et al, Signal Transduction and Targeted Therapy 2021*).*

Lithium inhibits GSK-3, thereby improving the activity of BDNF (brain-derived neurotrophic factor) as shown *in vitro* and *in vivo.* The role of lithium in increasing BDNF expression plus the role of BDNF in neuron survival has led to suggest that lithium plays a role in the treatment of neurodegenerative diseases. The Wnt signaling pathway is implicated in neurodegenerative diseases and cancer. Lithium inhibition of GSK-3 has been shown to specifically inhibit the Wnt signaling pathway (Towards a unified understanding of Lithium action, Topical Review, 2017, 586).

Lithium inhibits GSK3β, and thus inhibits the assembly and activation of the NLRP3 inflammasome, cell death and IL-1β and IL-18 pro-inflammatory cytokines release as seen in vivo and in vitro. Lithium treatment, through inhibition of GSK3β, prevents reactive oxygen species (ROS) production, thereby prevents the NLRP3 inflammasome activation in a mouse model of ischemic stroke and spinal cord injury, and exerts anti-inflammatory and neuroprotective effects (B Chen et al Commun Biol 2022*;* YJ Zhao et al Neural Regen Res 2022).

NLRP3 is an intracellular sensor. ATP-P2X7 receptor binding activates the potassium channel TWIK2 which induces potassium efflux. A drop in intracellular potassium levels allows NLRP3 structure modification leading to its activation. Potassium treatment or potassium efflux inhibition prevents NLRP3 inflammasome assembly and activation in in vitro mouse and human cells (β).

Magnesium is also involved in NLRP3 inflammasome activation as described in vitro and in vivo. Indeed, magnesium deficiency leads to NLRP3-induced pyroptosis by gasdermin D cleavage. On the other hand, magnesium supplementation inhibits calcium influx-dependent gasdermin D activity, leading to pyroptosis (D Wang et al Cell Death Differ 2020*;* C Li et al Acta Biochimica and Biophysica Sinica 2021).

Preventive treatment with lithium, by inhibition of GSK3β, prevents the increase in taxol-induced GSK3β activity in rats, which simultaneously reduces AKT (protein kinase B) and mTOR (mechanistic target of rapamycin) activities, thereby preventing the development of taxol-induced neuropathic pain (Inhibition of glycogen synthase kinase 3beta activity with lithium prevents and attenuates paclitaxel-induced neuropathic pain, Neuroscience. 2013 December 19; 254*).*

Lithium has anti-inflammatory properties that induce the reduction of both pro-inflammatory cytokines and interleukin TNF-alpha. On the other hand, lithium regulates the biosynthesis of different neurotransmitters and / or associated receptors such as serotonin and glutamate. Lithium has an anti-allodynia effect as well as a stimulating effect on the production of cerebral beta-endorphins, a MOR agonist with strong analgesic properties. (Lithium reverses mechanical allodynia through a µ opioid-dependent mechanism, Molecular Pain 2018 Volume 14: 1-8)

Lithium also contributes to calcium homeostasis and blocks calcium-dependent activation of pro-apoptotic signaling pathways that confirms its cytoprotective action (Molecular actions and therapeutic potential of lithium in preclinical and clinical studies of CNS disorders, Pharmacol Ther. 2010 November; 128(2): 281-304)*.*

Because the Li⁺ ion inhibits the GSK-3 enzyme which is an important enzyme at the crossroads of several signaling systems, it impacts multiple downstream targets, including: ionotropic glutamate signaling, multiple transcription factors, and the Wingless (Wnt)/β catenin integration pathway. Wnt signaling plays a role in structural brain processes such as neuronal development, synapse formation, and neuronal plasticity (LITHIUM IN THE TREATMENT OF BIPOLAR DISORDER: PHARMACOLOGY AND PHARMACOGENETICS, Mol Psychiatry. 2015 June; 20(6): 661-670*).*

The two-metal-phosphate complex ATP-Mg-Li is formed at normal concentrations of ATP and Mg²⁺ found in plasma or cytoplasm. In addition, high levels of ATP corresponding to biological activity or in response to stress in the cytoplasm, organelles (e.g. mitochondria), and extracellular matrix serve as potential reservoirs for accumulating lithium ions (Li⁺). The two-metal phosphate complex ATP-Mg-Li is found to be the bioactive form of lithium acting by co-binding with Mg²⁺ to phosphates possessing ligands or receptor cofactors or enzymes. The ATP-Mg-Li complex makes it possible that lithium ions can act by modulating the normal function of ATP as a ligand of cell surface purine receptors, of which there are two subtypes: P2X, which are ion channels that mediate the influx of extracellular calcium(Ca²⁺) ions into the cytoplasm, and P2Y, which are coupled to G receptor proteins (GPCRs) that activate the second messenger pathway of inositol trisphosphate to release calcium ions from intracellular reserves, to modulate signaling in the central nervous system and periphery. (A Molecular Model for Lithium's Bioactive Form, Biophysical Journal 111, 294-300, July 26, 2016).

One of the main limitations of lithium treatments is its toxicity. Indeed, research on bipolar treatment is strongly focused on identifying alternatives to lithium carbonate salt used to reduce toxicity and improve the therapeutic window. One of the challenges in using lithium in the treatment of diseases with an inflammatory base is therefore to widen the therapeutic window currently available.

Potassium is also an essential element. It is the most abundant cation in intracellular fluid, where it plays a key role in maintaining cellular function, especially in excitable cells such as muscles and nerves. Potassium deficiency is associated with glucose intolerance and diabetes. The interaction of K⁺ with extracellular Na⁺ increases the activation energy for water movement promoting ionic exchanges and balances, especially those of lithium (Potassium Intake, Bioavailability, Hypertension, and Glucose Control, Nutrients 2016, 8, 444).

Deregulation of the activity of these three ions is involved in neurogenic inflammation also called neuroinflammation, "low-noise inflammation" or "inflammaging" (inflammation related to aging), including chronic inflammation, involved for example in fibromyalgia, neuropathy, osteoarthritis, polyarthritis, metabolic syndrome, diabetes, obesity, atherosclerosis, irritable bowel syndrome, ulcerative colitis, Crohn's disease (inflammatory bowel disease), multiple sclerosis, Alzheimer's disease, Parkinson's disease, Huntington's disease, Charcot-Marie-Tooth disease, amyotrophic lateral sclerosis, migraine, asthma, pain, or in inflammatory reactions with acute components, involved for example in stroke, systemic inflammatory response syndrome, infarction, sepsis, burns, Covid, trauma or post-operative inflammation.

These deregulations are impactful, individually and in conjunction.

A water-based mineral composition comprising potassium, sodium, calcium, magnesium, silicon oxide, lithium, strontium, iron, manganese, copper, lead, zinc, aluminum, fluorine, chloride ions and sulfate ions has been shown to be useful in the treatment of metabolic diseases through reduction of pro-inflammatory cytokines (KR20200109179).

A simultaneous balanced approach is justified in order to provide a therapeutic response to inflammation and its physiological repercussions such as cell degeneration.

It has been discovered by the present inventors that a composition containing a particular mixture of lithium, magnesium and potassium, in specific molar ratios, allows, and it alone, to prevent and / or treat inflammation particularly effectively, especially in oral, parenteral or ocular administration.

### Solution of the invention

The present invention is defined by the appended claims. Any subject-matter falling outside the scope of the claims is provided for information only.

It is a multi-ionic composition for use in the treatment or prevention of inflammation comprising mineral and/or organic salts, including at least one lithium, magnesium and potassium salt, characterized by the following molar ratios: lithium 1 - magnesium [0.13 - 0.34] - potassium [1.20-2.40], wherein said treatment or prevention comprises oral or parenteral administration of said multi-ionic composition.

Preferably, the lithium concentration in the composition is between 0.01 and 10 mmol / kg.

Preferably, the molar ratio of magnesium to lithium is [0.14 - 0.32], preferably [0.15 - 0.30], preferably [0.18 - 0.25].

Preferably, the molar ratio of potassium to lithium is [1.30 - 2.30], preferably [1.35 - 2.15], preferably [1.40 - 2.15], preferably [1.55-1.75], preferably [1.58-1.72], preferably again [1.61-1.70].

This multi-ionic complex consists of a novel combination of "magnesium - lithium - potassium" according to a specific molar ratio algorithm. The adaptation of one of the components, depending on the therapeutic response to inflammation, results in the proportional adaptation of the concentrations of the other two elements.

This novel algorithmic combination aims, in particular by oral or parenteral administration, at simultaneously breaking the pro-inflammatory vicious circle that feeds itself, at restoring cellular homeostasis in these ions that is found altered in many pathologies, at promoting dynamic competition for the binding sites of these ions in order to obtain corrective physiological responses (enzymatic, metabolic, neurotransmissions...), and at generating a potentiated synergistic response that will strengthen the cellular response to insult in order to manage inflammation quickly and sustainably. It is also demonstrated below that the combination of these three elements significantly reduces the toxicity of lithium (compared to lithium taken alone) and allows its use in the treatment of bipolar disorder to be considered.

The action of the multi-ionic complex promotes the restoration of homeostasis altered by the inflammatory reaction, in particular that of magnesium and its ionic form Mg²⁺, as well as potassium and its ionic form K⁺ whose concentrations can be significantly impacted, and helps to restore optimal cellular physiological function, improve cellular metabolism and restore cytoprotective, as well as anti-apoptosis activity at the neuronal level.

Its action promotes the rebalancing of potassium and restores its key role in maintaining cellular function, especially in excitable cells such as nerve cells. In addition, it promotes the interaction of potassium with sodium, in ionic form K⁺ with Na⁺ extracellular and increases fluid movements promoting ionic exchanges and balances, especially those of lithium. Lithium has indeed a strong biological activity, as it does not have its own system responsible for regulating its concentration which can vary significantly in intra- and extra-cellular fluids, its external contribution must be adapted according to the therapeutic need, coordinated and weighted.

The bioactive multi-ionic complex brings together lithium, magnesium and potassium, which exert their multiple physiological and biochemical effects, described above.

Lithium combines its important anti-inflammatory properties with regulatory actions of the biosynthesis of neurotransmitters such as serotonin and glutamate, a stimulating effect on the production of brain beta-endorphins reducing pain.

Lithium and magnesium are linked. The two-metal phosphate complex ATP-Mg-Li is found to be the bioactive form of lithium acting by co-binding with Mg²⁺ to phosphates possessing ligands or receptor cofactors or enzymes. This two-metal-phosphate complex forms at normal concentrations of ATP and Mg²⁺ found in plasma or cytoplasm. In addition, in stressful situations that will raise ATP levels in the cytoplasm, organelles such as mitochondria, the extracellular matrix will serve as potential reservoirs to accumulate Li⁺.

Only the specific combination of molar ratios according to the invention ensures an absence of cellular toxicity, thus ensuring compliance with tissue homeostasis, especially that of more sensitive nerve tissues, and allows to obtain significant effectiveness for the prevention and / or treatment of neurogenic inflammation. Only the compositions meeting the definition according to the invention allow in particular a protective action of neurons, a protective and restorative action of neurites (axon-dendrites) and myelin sheaths against damage caused by cisplatin, treatment recognized as inducer of neurogenic inflammation. Without prejudging the mechanisms underlying the obtaining of such a surprising effect, it can be thought that only this combination of lithium, magnesium and potassium, in the molar ratios according to the invention, makes it possible to take maximum advantage of the biological activities of each of these elements, in the complex environment of the human and animal organism, involving both inhibitory effects at the level of the pro-inflammatory vicious circle, a restoration of cellular homeostasis in these ions that are found altered, especially that of magnesium, and its ionic form Mg²⁺ essential for the optimal functioning of mitochondria, as well as potassium and its ionic form K⁺, a dynamic competition effect at the level of ion channels as well as for the sites of fixation of these ions, associated with an increase in intra- and extracellular fluid movements to reduce lithium toxicity, in particular, activation of immune, enzymatic, metabolic and neurotransmission responses, a potentiated synergistic response that will strengthen resistance and cellular response to insult by cytoprotective action, restoration of neuronal excitability potential, as well as deactivation of apoptosis pathways activated by extracellular ATP.

Lithium is present in the composition at a concentration between 0.01 and 100 mmol/kg. The lithium concentration in the composition is preferably between 0.1 and 50 mmol/kg, preferably between 0.2 and 25 mmol/kg, for example between 0.5 and 15 mmol/kg, or between 1 and 6 mmol/kg. Ideally, the targeted cells are not effectively exposed to concentrations greater than 10 mmol/kg, at the risk that the toxicity outweighs the benefit for the treatment of inflammation. Nevertheless, in the presence of magnesium and potassium, it is shown below that the toxicity of lithium is significantly mitigated compared to the toxic concentrations generally described. Below 0.01 mmol/kg of lithium, the effectiveness of the composition is compromised.

Preferable, lithium is present in the composition at a concentration between 0.01 and 100 mmol/kg. The lithium concentration in the composition is preferably between 0.1 and 50 mmol/kg, preferably between 0.2 and 25 mmol/kg, for example between 0.5 and 15 mmol/kg, or between 1 and 6 mmol/kg.

In the context of oral or parenteral use/administration, the composition of the invention simultaneously aims to restore cellular homeostasis in these ions, in particular Mg²⁺, as well as potassium and its ionic form K⁺, to promote dynamic competition for binding sites, and to generate a potentiated synergistic response. The composition of the invention could also be administered ocular route, for example in the form of eye drops.

The composition of the invention is useful in the prevention and/or treatment of inflammation, including chronic inflammation or acute inflammation.

Neurogenic inflammation also called neuroinflammation, "low-noise inflammation" or "inflammaging" (age-related inflammation), including chronic inflammation, involved for example in fibromyalgia, neuropathy, osteoarthritis, polyarthritis, metabolic syndrome, diabetes, obesity, atherosclerosis, irritable bowel syndrome, ulcerative colitis, Crohn's disease (inflammatory bowel disease), multiple sclerosis, Alzheimer's disease, Parkinson's disease, Huntington's disease, Charcot-Marie-Tooth disease, amyotrophic lateral sclerosis, migraine, asthma, pain, or in inflammatory reactions with acute components, implicated for example in strokes, systemic inflammatory response syndrome, infarction, sepsis, burns, Covid, trauma or post-operative inflammation.

The release of inflammatory markers such as TNF-α, IL-1β, IL-18 and/ or NFκB or the activation of enzymes such as Caspase are recurrent in all these pathologies, underlying an inflammatory cause. The composition of the invention makes it possible to inhibit the release of these markers or the activation of these enzymes, in a preventive context as well as in a curative context. The composition of the invention can be used for the prevention and/or treatment of inflammation orally or parenterally.

Preferably, the composition of the invention is for the treatment and or prevention, by oral or parenteral administration, of the inflammation associated with Alzheimer's disease and Huntington's disease.

Preferably, the composition of the invention is for the treatment and/or prevention orally or parenterally of the inflammation associated with strokes.

At least one mineral or organic salt of lithium, magnesium and potassium means that these species are present in ionic form. The cations Li⁺, Mg²⁺ and K⁺ are associated with one or more anions, i.e. they can come from mixed salts and/or each from a different salt.

Advantageously, lithium is introduced into the composition in the form of lithium chloride, lithium hydroxide, lithium carbonate, lithium citrate, lithium gluconate, lithium orotate and/or any other pharmaceutically acceptable salt.

Preferably, for oral or parenteral use, the composition comprises lithium chloride.

Advantageously, magnesium is introduced into the composition in the form of magnesium chloride, magnesium carbonate, magnesium hydroxide, magnesium oxide, magnesium sulfate, magnesium silicate, magnesium bisglycinate, magnesium malate, magnesium glycerophosphate, magnesium stearate, magnesium ascorbate, magnesium taurate, magnesium citrate, magnesium gluconate, magnesium taurinate, and/or any other pharmaceutically acceptable mineral or organic salt.

Preferably, for oral or parenteral use, the composition comprises magnesium chloride, optionally hydrated.

Advantageously, potassium is introduced into the composition in the form of potassium chloride, potassium bromide, potassium iodide, potassium phosphate, potassium carbonate, potassium hydroxide, potassium silicate, potassium gluconate, potassium citrate, potassium malate, potassium glycerophosphate, potassium lactate, potassium pidolate, potassium aspartate, and/or any other pharmaceutically acceptable mineral or organic salt. Preferably, for oral or parenteral use, the composition comprises potassium chloride.

The composition of the invention may comprise other mineral or organic salts.

The composition may for example comprise at least one silicon salt, preferably a silicate such as sodium silicate (Na₂SiO₃), optionally hydrated, potassium silicate orthosilicic acid, monomethylsilanetriol and / or any other pharmaceutical or organic salt pharmaceutically acceptable, especially for oral or parenteral use.

Silicon is a co-factor of prolyl hydroxylase that participates in the stimulation of fibroblasts. It decreases the permeability of capillaries, has an anti-edematous, soothing and refreshing effect.

It plays a role in the regulation of the cell cycle of lymphocytes which ultimately affects the immune and inflammatory response.

Silicon decreases the level of expression in inflamed areas of endothelial nitric oxide synthase (eNOS), vascular endothelial growth factor (VEGF), fibroblast growth factor (FGF), epidermal growth factor (EGF), kappa-activated B-cell light chain nuclear factor (NF-κB), various cytokines (TNF-α and IL-1β).

Depending on the inflammation underlying the pathology envisaged, the proportion of silicon in relation to other salts is adapted.

The composition may for example comprise at least one manganese salt, such as manganese chloride, manganese sulfate, manganese carbonate, manganese citrate, manganese malate, manganese glycerophosphate, manganese lactate, manganese pidolate, manganese aspartate, manganese gluconate, manganese alginate, manganese picolinate and / or any other pharmaceutically acceptable mineral or organic manganese salt, especially in oral or parenteral use.

Manganese is involved in tissue physiology and biology through its role as a cofactor in many enzymatic processes. Manganese plays an essential role in the regulation of glucose tolerance by acting synergistically with magnesium, which allows in particular the reduction of nervous and epidermal metabolic stress.

The composition may for example comprise at least one mineral or organic salt of zinc, such as zinc citrate, zinc orotate, zinc sulfate, zinc citrate, zinc malate, zinc glycerophosphate, zinc lactate, zinc pidolate, zinc aspartate, zinc gluconate which has an important role in many vital enzymatic processes such as DNA and protein synthesis, wound healing, insulin metabolism, development and proper functioning of the nervous system...

The composition may for example comprise at least one mineral or organic salt of copper, such as copper carbonate, copper citrate, copper malate, copper glycerophosphate, copper lactate, copper pidolate, copper aspartate, copper gluconate. Copper is the constituent of several enzymes, which are involved in the metabolism of carbohydrates, lipids and iron. It has an antioxidant role.

An oral or orally administered composition is a composition intended to be ingested. Preferably, the composition is in the form of tablet, pill, capsule, solution, syrup, lyophilizate, that is, it may contain excipients necessary for the desired formulation.

A parenteral composition or parenterally administered means a composition injectable next to the digestive tract, intravenously (injection via a hypodermic needle a catheter, or a cath port), subcutaneously, intramuscularly or intraperitoneally. In the case of a parenteral composition, it is in liquid form.

In the case of a parenteral composition, the mineral or organic salts of the composition of the invention are dissolved in water, that is to say that the main solvent of the composition is preferably water. The water represents at least 50% by weight of the composition, preferably at least 75% by weight, at least 80%, and preferably at least 90% or 95%. The composition may comprise other liquid ingredients, in particular to promote stability and biocompatibility (pH, plasma osmolarity, natremia), considered useful by those skilled in the art.

The composition of the invention may also comprise other molecules, additives or excipients, for example preservatives, antibiotics, stabilizing agents, thickeners, antioxidants. These additives may be useful for the preservation of the composition of the invention.

The composition may for example comprise one or more vitamins, such as vitamin E. Vitamin E is a powerful antioxidant and modulates immune function.

The invention relates to the use of the composition as a medicine, for human or veterinary use.

Neurogenic inflammation also called neuroinflammation, "low-noise inflammation" or "inflammaging" (age-related inflammation), including chronic inflammation, involved for example in fibromyalgia, neuropathy, osteoarthritis, polyarthritis, metabolic syndrome, diabetes, obesity, atherosclerosis, irritable bowel syndrome, ulcerative colitis, Crohn's disease (inflammatory bowel disease), multiple sclerosis, Alzheimer's disease, Parkinson's disease, Huntington's disease, Charcot-Marie-Tooth disease, amyotrophic lateral sclerosis, migraine, asthma, pain, or in inflammatory reactions with acute components, implicated for example in strokes, systemic inflammatory response syndrome, infarction, sepsis, burns, Covid, trauma or post-operative inflammation.

The invention will be better understood using the following description, with reference to the figures, illustrating:
Fig. 1: effect of the compositions of the invention on the survival of cortical neurons after glutamate injury;
Fig. 2: effect of the compositions of the invention on the release of TNF-α after cisplatin injury;
Fig. 3: effect of the compositions of the invention on the release of IL-1β after cisplatin injury;
Fig. 4: effect of the compositions of the invention on the level of Caspase-3 in the Schwann cells after cisplatin injury;
Fig.5: effect of the compositions of the invention on the survival of dopaminergic neurons after MPP injury;
Fig.6: effect of the compositions of the invention on the survival of cortical neurons after oxygen deprivation and glucose;
Fig.7: anti-inflammatory effect of compositions of the invention in vitro on human mononuclear blood cells stimulated by LPS;
Fig.8: anti-inflammatory effect of single ions in vitro on human mononuclear blood cells stimulated by LPS;
Fig.9: anti-inflammatory effect of 3 algorithmic variations of compositions of the invention in vitro on human mononuclear blood cells stimulated by LPS;
Fig.10: effect of the compositions of the invention on the anti-inflammatory properties in an in vivo model of DSS-induced ulcerative colitis.

The algorithmic combination was tested *in vitro* and *in vivo* on various models known to be significant inflammatory diseases described above.

### Example 1 - preparation of solutions at various concentrations for in vitro tests.

Solutions comprising lithium chloride, magnesium chloride and potassium chloride were prepared in reverse osmosis water.

They are shown in Table 1 below.

| **TA64** | **3** | **5** | **9** | **12** | **15** | **19** | **22** |
|---|---|---|---|---|---|---|---|
| Li mg/kg | 2, 0 | 4, 5 | 6, 0 | 9, 0 | 12,0 | 15,0 | 18, 0 |
| Li mmol/kg | 0, 3 | 0, 7 | 0, 9 | 1, 3 | 1, 7 | 2, 2 | 2, 6 |
| Li ratio | 1, 0 | 1, 0 | 1, 0 | 1, 0 | 1, 0 | 1, 0 | 1, 0 |
| Mg mg/kg | 1, 5 | 3, 5 | 4, 6 | 6, 9 | 9, 2 | 11,5 | 13,8 |
| Mg mmol/kg | 0, 1 | 0, 1 | 0, 2 | 0, 3 | 0, 4 | 0, 5 | 0,6 |
| Mg ratio | 0, 2 | 0, 2 | 0, 2 | 0, 2 | 0, 2 | 0, 2 | 0, 2 |
| K mg/kg | 18,3 | 41,3 | 55,0 | 82,5 | 110,0 | 137,5 | 165,0 |
| K mmol/kg | 0,5 | 1,1 | 1,4 | 2,1 | 2,8 | 3,5 | 4,2 |
| K ratio | 1, 6 | 1, 6 | 1, 6 | 1, 6 | 1, 6 | 1, 6 | 1, 6 |

**Table 1**

| **TA64** | **26** | **28** | **40** | **96** | **128** |
|---|---|---|---|---|---|
| Li mg/kg | 21,0 | 24,0 | 40, 0 | 72,0 | 96 |
| Li mmol/kg | 3, 0 | 3, 5 | 5, 8 | 10,4 | 13,9 |
| Li ratio | 1, 0 | 1, 0 | 1, 0 | 1, 0 | 1, 0 |
| Mg mg/kg | 16,1 | 18,4 | 30, 6 | 55,4 | 73.9 |
| Mg mmol/kg | 0, 7 | 0, 8 | 1, 3 | 2,3 | 3, 1 |
| Mg ratio | 0, 2 | 0, 2 | 0, 2 | 0, 2 | 0, 2 |
| K mg/kg | 192,5 | 220,0 | 366,7 | 660,0 | 880, 0 |
| K mmol/kg | 4, 9 | 5, 6 | 9, 4 | 16,9 | 22,6 |
| K ratio | 1, 6 | 1, 6 | 1, 6 | 1, 6 | 1, 6 |

### Reference Example 2 - effect of the compositions of the invention on an Alzheimer's model of glutamate injury on cortical neurons (cortical neurons)

Rat cortical neurons are cultured according to the procedure described by Singer (1999).

Female rats with 15 days gestation were killed by cervical dislocation (Wistar rats; January Lab) and fetuses were removed from the uterus. Embryonic cortices were collected and placed in an icy medium of Leibovitz 15 (L15; PanBiotech, Ref P04-27055, Batch: 3870221) containing 2% Penicillin-Streptomycin (PS; PanBiotech, ref: P06-07100, Batch: 5870421) and 1% bovine serum albumin (BSA; PanBiotech, ref: P06-1391100, lot: H210207). The cortices were dissociated by trypsinization for 20 minutes (min) at 37°C (Trypsin EDTA 1X; PanBiotech, Ref: P10-023100, Batch: 3030221). The reaction was halted by the addition of Dulbecco's modified Eagle medium (DMEM; PanBiotech, Ref: P04-03600, Batch: 1130721) containing DNase I grade II (0.1 mg/ml; PanBiotech, Ref: P60-37780100, Batch: H181015) and 10% fetal calf serum (FCS; Invitrogen, Ref: 10270106, Lot: 2275115). The cells were then mechanically dissociated by 3 passes through a 10 ml pipette. The cells were then centrifuged at 515 x g for 10 min at +4°C. The supernatant was discarded and the cell pellets were resuspended in a defined culture medium consisting of Neurobasal (Invitrogen, Ref: 11570556, Batch: 2327396) supplemented with 2% B27; (Invitrogen, Ref: 11530536, Batch: 2337321), L-glutamine (2 mM; PanBiotech, Ref: P04-80100, Batch: 2770620), 2% PS and 10ng/mL BDNF (Peprotech, Ref: 450-02, Batch: 102061). The viable cells were then counted in a Neubauer cytometer using the trypan blue exclusion test. The cells were seeded to a density of 30000 cells/wells in 96-well plates (pre-coated with poly-D-lysine; Greiner, Ref: 655940, Lot: E200933A) and were grown at 37°C in humidified air (95%/CO2 (5%) atmosphere. Half of the middle was changed every 2 days with cool medium.

A glutamate solution (Sigma, Ref: G1501, Batch SLBD7305V) is reconstituted in a culture medium at 10 mM. A control solution is prepared under the same conditions.

After 11 days of culture the primary cortical neurons are pretreated for one hour with the compositions of Example 1 or the reference compound (BDNF, 50 ng/mL) and then intoxicated with glutamate at a concentration of 40 µM for incubation of 20 minutes.

The following conditions were tested:
- Control medium
- Control + Glutamate (40uM, 20 min)
- Composition TA6496 + Glutamate (40uM, 20 min)
- Composition TA6440 + Glutamate (40uM, 20 min)
- Composition TA6415 + Glutamate (40uM, 20 min)
- BDNF (50 ng/mL) + Glutamate (40uM, 20 min)

A culture was carried out with 6 wells per condition.

Primary endpoint: measurement of the total number of cortical neurons.

After 24 hours of incubation, the cells were fixed with a 4% solution of paraformaldehyde (Alpha Aesar; Ref: J19943, Batch: 206909) for 15 min at room temperature. The cells were then permeabilized with saline phosphate buffer (PBS; PanBiotech; ref: P04-36500, Batch: 3410921) containing 0.1% of triton X-100 (Sigma; ref: T9284, Batch: 118K01602) for 30 min at room temperature. Non-specific sites were saturated with saline phosphate buffer solution containing 3% BSA and 3% FCS for 1h. The cells were then incubated with a chicken polyclonal anti-MAP-2 antibody (1/2000, Abcam; ref: ab5392, Batch: GR3405939-2) in a saturating buffer for 12h at 4°C.

These antibodies were revealed with an Alexa Fluor 568 goat anti-chicken IgG (1/400, Molecular Probe, ref: A110041, lot: 1963088) in saturating buffer for 1 hour at room temperature. The cell nuclei were labeled with a fluorescent marker (Hoechst solution, Sigma; ref: B1155, lot: 046M4048V) in the same solution.

For each condition, 20 pictures per well were taken using InCell AnalyzerTM 2200 (GE Healthcare) with 20x magnification. The images of each crop well were taken under the same conditions. The automatic analysis of the number of cortical neurons was performed using the Developer software (GE Healthcare). A total of 6 data per experimental condition was provided.

### Statistics:

Data were expressed on average ± s.e. (out of 6 data per condition, 1 culture). An overall analysis of the data was performed using a one-way analysis of variance (ANOVA) followed by a Dunnett test. The significance level is set at p <0.05.

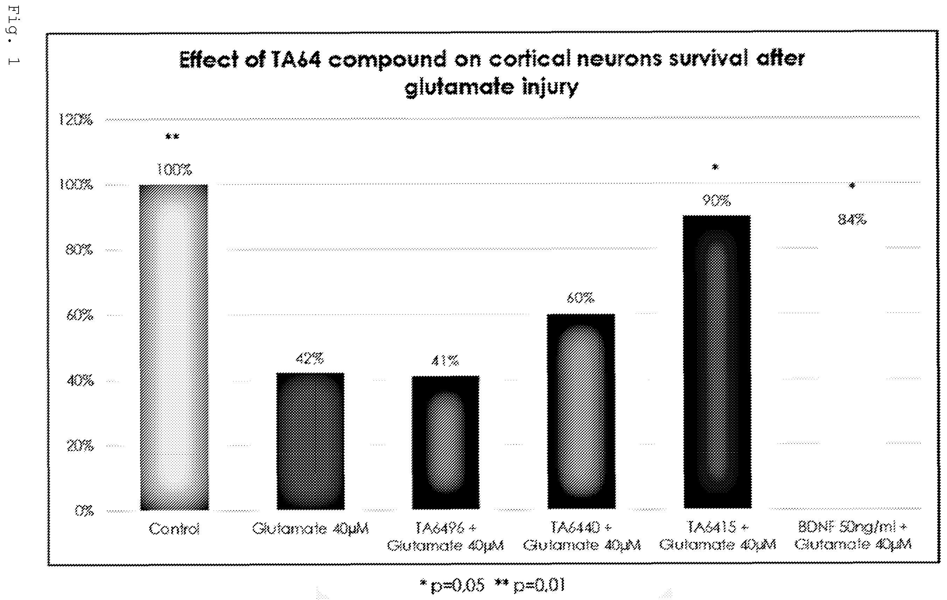

The results are shown in Fig 1. The composition TA6415 significantly protects cortical neurons from apoptosis after glutamate injury, at a level slightly above BDNF control.

### Example 3 - anti-inflammatory effect of the compositions of the invention on the release of TNF-α and IL-1β in sensory neurons.

Sensory neurons are part of the peripheral nervous system. Their cell bodies are located in the Dorsal Root Ganglia (D.R.G.) along the spinal cord and emit extensions to the most distal extremities. One of the properties of sensory neurons is their ability to regenerate their extensions after cutting nerves. This property is related to the presence of Schwann cells, nourishing and myelinating cells of the peripheral nervous system that release specific growth factors for axon growth.

The in vitro model used is a culture of sensory neurons myelinated by Schwann cells (Callizot et al.,2011, Exp Cell Res 317:2374-2383). This model, miniaturized in 96 wells, makes it possible to analyze the effects of molecules on the development of sensory neurons and on the myelination of axons by Schwann cells.

The objective of this study is to investigate the effect of TA64 compositions at 6 different concentrations on the release of TNF-α and IL-1β by sensory neurons after cisplatin injury.

### Protocol for the co-culture of sensory neurons and Schwann cells:

Female rats with 15 days gestation were killed by cervical dislocation (Wistar rats; January Lab) and fetuses were removed from the uterus. Embryonic DRGs were collected and placed in an icy medium of Leibovitz 15 (L15; PanBiotech, Ref P04-27055, Batch: 5331021) containing 2% Penicillin-Streptomycin (PS; PanBiotech, ref: P06-07100, Batch: 9300621 ) and 1% bovine serum albumin (BSA; PanBiotech, ref: P06-1391100, lot: H210207). DRG were dissociated by trypsinization for 20 minutes (min) at 37°C (Trypsin EDTA 1X; PanBiotech, Ref: P10-023100, Batch: 8600621). The reaction was halted by the addition of Dulbecco's modified Eagle medium (DMEM; PanBiotech, Ref: P04-03600, Batch: 1130721) containing DNase I grade II (0.1 mg/ml; PanBiotech, Ref: P60-37780100, Batch: H181015) and 10% fetal calf serum (FCS; Invitrogen, ref: 10270106, lot: 2319479). The cells were then mechanically dissociated by 3 passes through a 10 ml pipette. The cells were then centrifuged at 180 x g for 10 min at + 4°C on a layer of BSA (3.5%) in medium L15. The supernatant was discarded and the cell pellets were resuspended in a defined culture medium consisting of Neurobasal (Invitrogen, Ref: 21103049, Batch: 2348949) supplemented with 2% B27; (Invitrogen, ref: 17504-044, Batch: 2336991), L-glutamine (2 mM; PanBiotech, Ref: P04-80100, Batch: 2770620), 2% PS and 50ng/mL NGF (Sigma, Ref: N1408, Lot: SLCG2596). The viable cells were then counted in a Neubauer cytometer using the trypan blue exclusion test. The cells were seeded to a density of 12000 cells/wells in 96-well plates (pre-coated with poly-D-lysine; Greiner, Ref: 655940, Lot: E200933A) and were grown at 37°C in humidified air (95%/CO2 (5%) atmosphere. Half of the middle was changed every 2 days with fresh medium. The cells are maintained for 7 days to allow the proliferation of Schwann cells and sensory neurons. On day 8, the medium is supplemented with 50 µg/mL of ascorbic acid (AA; Sigma, Ref 092902, Batch: 05316HJ-438) to initiate the differentiation of basal Schwann cells into myelinating Schwann cells.

Under these conditions, after 5 days of culture with AA, myelin sheaths are detected with an anti-MAG (Myelin antigen glycoprotein) antibody, an early marker of myelin.

### Preparation, exposure and drug treatment with cisplatin: curative protocol

Cis-diammineplatinum(II) dichloride (cisplatin; Sigma, ref: P4394, lot: MKCN8054) was reconstituted in DMSO at 24 mM (stock solution) . The control environment was prepared under the same conditions. After 12 days of culture with AA, the primary sensory neurons were pretreated for 2 hours with the compositions of Example 1 or with a reference compound (NGF, 50ng/ML) and then intoxicated with cisplatin. The cisplatin preparation was used on neurons at a final concentration of 12 µM diluted in control medium for 24 hours of incubation.

The following conditions were tested:
- Control medium
- Control + Cisplatin (12uM, 24 h)
- Composition TA6426 + Cisplatin (12µM, 24 h)
- Composition TA6422 + Cisplatin (12µM, 24 h)
- Composition TA6419 + Cisplatin (12µM, 24 h)
- Composition TA6415 + Cisplatin (12µM, 24 h)
- Composition TA6412 + Cisplatin (12µM, 24 h)
- Composition TA6409 + Cisplatin (12uM, 24 h)
- Control + NGF (50 ng/mL) + Cisplatin (12uM, 24 h)

A culture was carried out with 6 wells per condition. After 6 h of incubation in the presence of cisplatin, the conditioned media are collected and stored at -80 ° C. The determination of TNF-α was carried out on the thawed medium by flow cytometry, according to the manufacturer's instructions (BD Bioscience, ref 561516, Batch: 0171683).

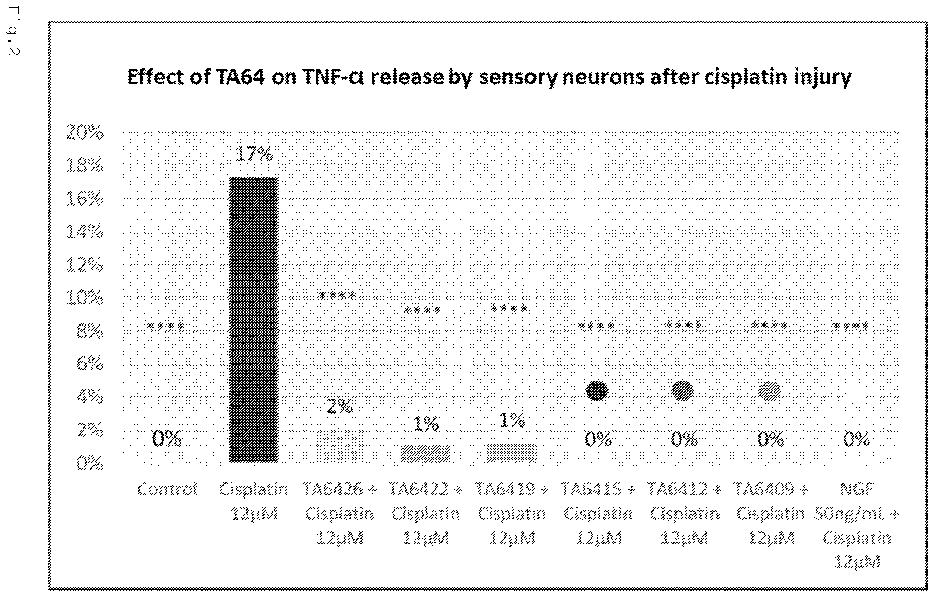

The results are shown in Fig.2.

After 6 hours of treatment with cisplatin, the conditioned media are removed and stored at -80 ° C. The determination by ELISA of rat IL-1β was performed on thawed samples, according to the manufacturer's instructions (Bio-techne, ref DY501, Lot P296813).

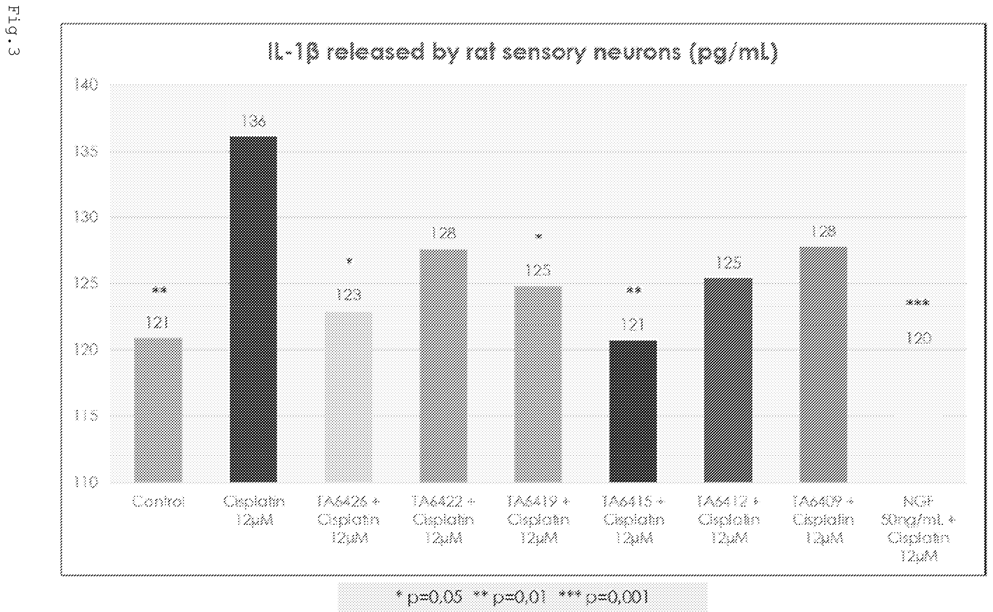

The results are shown in Fig.3

Data were expressed on average ± s.e. (out of 6 data per condition, 1 culture). An overall analysis of the data was performed using a one-way analysis of variance (ANOVA) followed by the Dunnett test. The significance level is set at p <0.05.

### Results:

Cisplatin at 12 µM induces an elevation of the release of TNF-α and IL-1β. All TA64 compositions tested significantly inhibit the release of TNF-α at the control level. The compositions TA6426, TA6419 and TA6415 significantly inhibit the release of IL-1β, while an overall trend is observable for all compositions tested.

### Reference Example 4 - anti-apoptosis effect of the compositions of the invention by analysis of caspase activation in sensory neurons (Alzheimer's model).

The protocol for the co-culture of sensory neurons and Schwann cells, as well as the preparation and exposure to cisplatin are the same as that detailed in Example 3.

The following conditions were tested:
- Control medium
- Control + Cisplatin (12uM, 24 h)
- Control +Composition TA6426 + Cisplatin (12uM, 24 h)
- Control +Composition TA6422 + Cisplatin (12uM, 24 h)
- Control +Composition TA6419 + Cisplatin (12µM, 24 h)
- Control +Composition TA6415 + Cisplatin (12µM, 24 h)
- Control +Composition TA6412 + Cisplatin (12µM, 24 h)
- Control +Composition TA6409 + Cisplatin (12uM, 24 h)

A culture was carried out with 6 wells per condition.

After 24 hours of treatment in the presence of cisplatin, the cells were fixed with a solution of acetic acid 5% (Sigma; ref: 33209; lot: SHBJ9236) and ethanol 95% (VWR; ref: 83813.360; lot: 20G074013) for 5 min at -20 °C, the control conditions were also set following the same procedure. The cells were then permeabilized and the non-specific sites were saturated with saline phosphate buffer (PBS; PanBiotech; ref: P04-36500, Batch: 5371021) containing 0.1% newt X-100 (Sigma; ref: T9284, Batch: 118K01602) for 30 min at room temperature. Non-specific sites were saturated with a PBS solution containing 3% BSA and 3% FCS for 1 hour. The cells were then incubated with a mouse anti-β-tubulin monoclonal antibody (1/2000, Sigma; ref: T8660, Batch: 034M4790V) and a rabbit cleaved anti-caspase-3 polyclonal antibody (1/500, Abcam, ref: ab13847, Batch: GR3286166-1) in a saturating buffer for 12 hours at +4°C. Antibodies were revealed with goat anti-mouse IgG Alexa Fluor 647 (1/400; Molecular Probe; ref : A212235 ; lot: 1922319) and an IgG Alexa Fluor 568 goat anti-rabbit (1/400; Molecular Probe; ref : A11011 ; Batch: 2192277) in a saturating buffer for 1 hour at room temperature. The cell nuclei were marked with a fluorescent marker (Hoeschst solution, Sigma, ref: B1155, Batch: 046M4048V) under the same conditions.

For each condition, 20 pictures per well were taken using InCell AnalyzerTM 2200 (GE Healthcare) with 20x magnification. The images of each crop well were taken under the same conditions. The number of sensory neurons and caspase-3 level were automatically assessed using Developer software (GE Healthcare). A total of 6 data per experimental condition was provided.

Data were expressed on average ± s.e. (out of 6 data per condition, 1 culture). An overall analysis of the data was performed using a one-way analysis of variance (ANOVA) followed by the Dunnett test. The significance level is set at p <0.05.

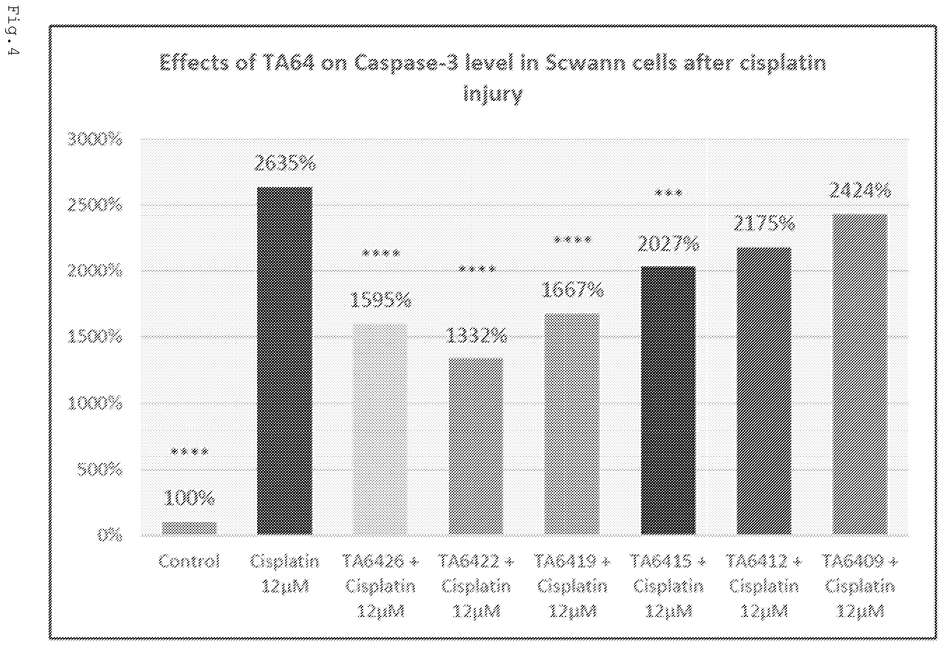

The results are shown in Figure 4.

The compositions TA6426, TA6422, TA6419 and TA6415 significantly reduce the release of caspase-3 in primary Schwann cells after cisplatin injury.

### Reference Example 5 - Effects of the compositions of the invention in an in vitro model of Parkinson's disease with MPP lesion (1-methyl-4-phenylpiridinium +) of mesencephalic neurons.

Rat dopaminergic neurons were cultured as described by Schinelli et al., 1988. In short, female rats with 15 days gestation were killed by cervical dislocation (Wistar rats; January Lab) and fetuses were removed from the uterus. The embryonic midbrains were collected and placed in an icy medium of Leibovitz 15 (L15; PanBiotech, Ref P04-27055, Batch: 3870221) containing 2% Penicillin-Streptomycin (PS; PanBiotech, ref: P06-07100, Batch: 1201220) and 1% bovine serum albumin (BSA; PanBiotech, ref: P06-1391100, lot: H200403). Only the ventral parts of mesencephalic flexure were used for cell preparation because this region of the developing brain is enriched with dopaminergic neurons. The midbrains were dissociated by trypsinization for 20 minutes at 37°C (Trypsin EDTA 1X; PanBiotech, Ref: P10-023100, Batch: 9821220). The reaction was stopped by the addition of Dulbecco's modified Eagle medium (DMEM; PanBiotech, Ref: P04-03600, Batch: 3090221) containing DNase I grade II (0.1 mg/ml; PanBiotech, Ref: P60-37780100, Batch: H181015) and 10% fetal calf serum (FCS; Invitrogen, Ref: 10270106, Lot: 2275115). The cells were then mechanically dissociated by 3 passes through a 10 mL pipette. The cells were then centrifuged at 180 x g for 10 min at +4°C on a layer of BSA (3.5%) in medium L15. The supernatant was discarded and the cell pellets were resuspended in a defined culture medium consisting of Neurobasal (Gibco, Ref: 21103049, Batch: 2242402) with 2% of B27 (Gibco, Ref: 17504044, Batch: 2295367), L-glutamine (2 mM; PanBiotech, Ref: P04-80100, Batch: 3301019), 2% PS, 10 ng/mL BDNF (PeproTech, Ref: 450-02, Batch: 071961) and 10 ng/mL glial-derived neurotrophic factor (GDNF; PanBiotech, ref: 450-10, lot: 0606B64). The viable cells were then counted in a Neubauer cytometer using the trypan blue exclusion test. The cells were seeded to a density of 40,000 cells/wells in 96-well plates (pre-coated with poly-D-lysine; Greiner, Ref: 655940, Batch: E20093UL) and were grown at 37°C in a humidified atmosphere (95%)/CO2 (5%). Half of the middle was changed every 2 days with fresh medium. Under these conditions, after 5 days of culture, astrocytes are present in the culture and release the growth factor allowing the differentiation of neurons. Under these conditions, 2 to 5% of neurons are dopaminergic neurons.

MPP+ (1-methyl-4-phenyl pyridinium), (Sigma, Ref: D048, Lot 0000047439) was reconstituted, in defined culture medium, at 10 mM (stock solution). The control environment was prepared under the same conditions. After 7 days of culture, the primary mesencephalic neurons were pretreated for 1 hour with test compounds or a reference compound (BDNF, 50ng/mL) and then intoxicated with MPP+ at a final concentration of 8µM for 48 hours of incubation to induce neuronal cell death of approximately 45% +/-5%.

The following conditions were tested:
- Control medium
- Control + MPP+ (16µM, 48 h)
- TA6496 + MPP+ (16µM, 48 h)
- TA6440 + MPP+ (16µM, 48 h)
- TA6415 + MPP+ (16µM, 48 h)
- BDNF (50 ng/mL) + MPP+ (16µM, 48 h)

After 48 hours of intoxication in the presence or absence of the compound to be tested, the cells were fixed with a solution of paraformaldehyde 4% (Alpha Aesar, ref J19943, Batch: 202585) for 20 min at room temperature, the control conditions were fixed according to the same procedure. The cells were then permeabilized and the non-specific sites were saturated with phosphate-buffered saline (PBS; PanBiotech; ref: P04-36500, Batch: 6300421) containing 0.1% saponin (Sigma; ref: S7900, Lot: BCBL8667V) and 1% FCS for 15 min at room temperature. The cells were incubated with an anti-Tyrosine Hydroxylase mouse monoclonal antibody (TH, 1/10,000, Sigma; ref: T1299, lot: 014M4835) in a PBS solution for 12 hours at 4°C. The antibody against TH marked dopaminergic neurons.

The staining was revealed by the addition of an Alexa Fluor 488 goat anti-mouse IgG (1/400, Molecular probe, ref: A11001, Batch: 2247988) in PBS with 1% FCS and 0.1% saponin for 1 hour at room temperature. Cell nuclei were labeled with a fluorescent marker (Hoechst, Sigma; ref: B1155, Batch: 046M4048V) in the same solution.

For each condition, 20 pictures per well were taken using InCell AnalyzerTM 2200 (GE Healthcare) with 20x magnification. Images of each crop well were taken in the same condition. The analysis of the cell bodies of the positive TH neurons was performed using Developer software (GE Healthcare). A total of 6 data per experimental condition were provided.

Data were expressed on average ± s.e. (out of 6 data per condition, 1 culture). An overall analysis of the data was performed using a one-way analysis of variance (ANOVA) followed by the Dunnett test. The significance level is set at p <0.05.

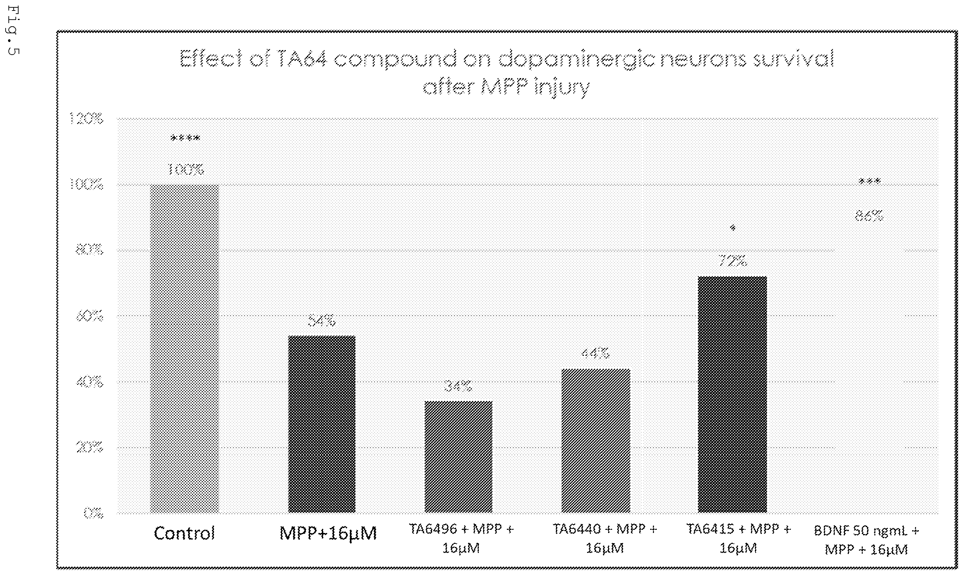

The results are shown in Figure 5.

TA6415 composition significantly protects dopaminergic neurons from apoptosis after MPP+ injury.

### Reference Example 6 - Effect of the compositions of the invention in a stroke model with oxygen deprivation and glucose.

Neuronal survival in a primary cortical culture of rats injured by oxygen deprivation / glucose (OGD) is studied here.

Stroke induces brain damage through the combined action of several mechanisms: including excitotoxicity, free radical production, inflammation and apoptosis. Excitotoxicity results from an excessive release of glutamate, the excitatory neurotransmitter of dead or dying neurons with inappropriate stimulation of N-methyl-D-aspartate (NMDA-R) receptors. High levels of glutamate trigger the death of neighboring neurons that have survived the initial aggression associated with a massive cell influx of Ca2+ but cannot survive such massive activation under restricted glucose and oxygen supply (Verleye et al., 2016).

The aim of this study is to investigate the protective effect of TA64 compounds, at 3 different concentrations, on neurons survival in a primary cortical culture of rats injured by oxygen/glucose deprivation (OGD), an in vitro stroke model.

Cortical cells were cultured as described in Example 2.

After 11 days of culture, primary cortical neurons were pretreated for 1 hour with TA64 or the reference compound (BDNF, 50 ng/mL).

Then the medium was removed and fresh glucose-free medium was added. This medium was composed of glucose-free DMEM (PanBiotech, Ref: P04-01549, Batch: 5471021) supplemented with 2% B27, 0.2 mM L-glutamine, 1% PS solution and 10 ng/mL BDNF. The cells were transferred to an anaerobic incubator with 95% N2 and 5% CO₂ at 37°C. After 2 hours, 25 mM of D-Glucose (Sigma, Ref: G8644, Batch: RNBJ8362) with or without compound TA64 were added to the culture medium and the cells were transferred to a conventional incubator with 95% air / 5% CO₂ at 37°C for 24 hours.

The following conditions were tested:
- Control (0.1% DMSO)
- Control + Oxygen and glucose deprivation (2 hours) and oxygen and glucose reperfusion (24 hours)
- Compound + Oxygen and glucose deprivation (2 hours) and oxygen and glucose reperfusion (24 hours)
- BDNF at 50ng/ml + oxygen and glucose deprivation (2 hours) and oxygen and glucose reperfusion (24 hours)

After 24 hours of intoxication, the cells were fixed with a solution of acetic acid 5% (Sigma, Ref: 33209, Batch: SHBJ9236) and ethanol 95% (VWR, Ref: 83813.360, Batch: 20G074013) for 5 min at -20 °C, the control conditions were also set following the same procedure. The cells were then permeabilized and the non-specific sites were saturated with saline phosphate buffer solution (PBS; PanBiotech; ref: P04-36500, Batch: 5371021) containing 0.1% saponin (Sigma; ref: S7900, Lot: BCBL8667V) and 1% FCS for 15 min at room temperature. The cells were incubated with an anti-MAP-2 mouse monoclonal antibody (1/5,000, Sigma; ref: M4403, lot: 035M4780V) in a PBS solution for 12 hours at 4°C. Dopaminergic neurons were labeled by anti-TH antibodies.

The staining was revealed by the addition of a goat anti-mouse IgG Alexa Fluor 568 (1/400, Molecular probe, ref: A11004, Batch: 2332536) in PBS with 1% FCS and 0.1% saponin for 1 hour at room temperature. The cell nuclei were labeled with a fluorescent marker (Hoechst, Sigma; ref: B1155, Batch: 046M4048V) in the same solution.

For each condition, 20 pictures per well were taken using InCell AnalyzerTM 2200 (GE Healthcare) with 20x magnification. The images of each culture well were taken under the same conditions. The analysis of the cell bodies of MAP-2-positive neurons was performed using Developer software (GE Healthcare). A total of 6 data per experimental condition were provided.

Data were expressed on average ± s.e. (out of 6 data per condition, 1 culture). An global analysis of the data was performed using a one-way analysis of variance (ANOVA) followed by the Dunnett test. The significance level is set at p <0.05.

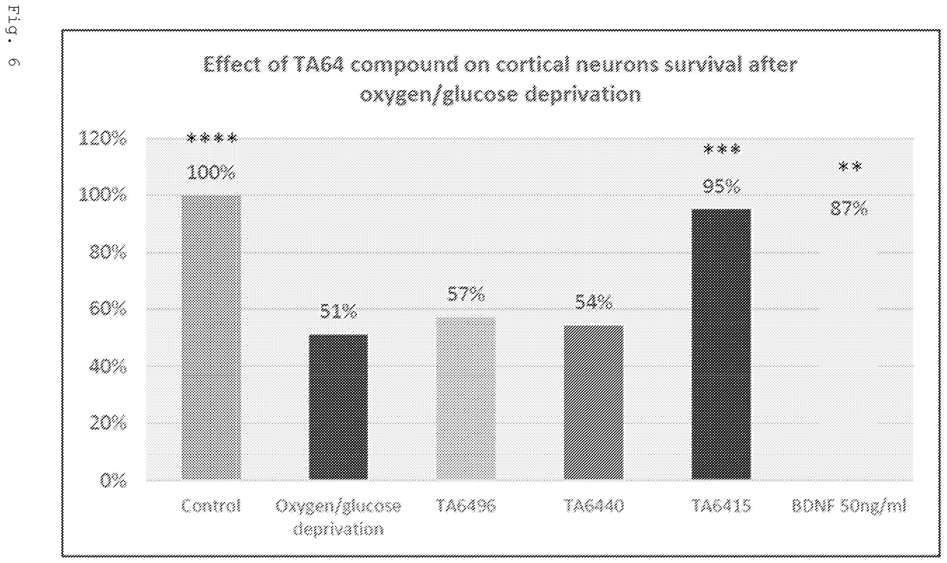

The results are shown in Figure 6.

The TA6415 composition significantly protects rat primary cortical neurons from apoptosis, even better than BDNF control at 50 ng/mL.

### Conclusions

The markers assessed in Examples 2, 3 and 4 are representative of Alzheimer's disease. The markers evaluated in Examples 3, 4 and 5 are representative of Parkinson's disease. The markers assessed in Examples 3, 4 and 6 are also involved in stroke.

It has thus been shown that the compositions according to the invention have a preventive and curative effect on these *models in vitro,* suggesting a significant action *in vivo.*

These markers, and in particular the release levels of TNF-α, IL-1β and caspase-3 are representative of inflammatory pathologies in general. It is therefore legitimate to consider a positive effect of the compositions of the invention on all inflammatory pathologies.

In addition to the anti-inflammatory activities of the compositions of the invention, the intrinsic toxicity of lithium, magnesium and potassium ions, isolated or in combination was also evaluated. It is repeated in the examples below.

### Reference Example 7 - Effect of Multi-ionic complexes on the length of neurites of sensory neurons

Treatment with the multi-ionic compositions TA6496, TA6440 and TA6415 for 24 hours on healthy cells, induces a slight but not significant increase in the length of neurites of sensory neurons (respectively 31%, 34% and 54% of the control).

Cisplatin at 12 µM induces a significant decrease in neurite length of sensory neurons (65% loss of neurites with p <0.01).

A treatment with the multi-ionic compositions according to the invention TA6440 and TA6415 is able to effectively preserve the length of neurites of sensory neurons from cisplatin lesions for 24 hours (respectively 19% with p <0.05 and 11% loss of neurites with p <0.05).

However, treatment with the multi-ionic composition TA6496 is able to partially but not significantly preserve the length of neurites of sensory neurons from cisplatin lesion for 24 hours (28% of neurite loss).

In parallel, the effect of isolated or combined ions two by two on the survival of sensory neurons and the length of neurites was also evaluated under the same conditions. The solutions are all prepared from chlorides of the ions tested.

The results are shown in Table 2.

**Table 2.**

| | | Average percentage of sensory neuron count relative to control | Average percentage of neurite length relative to control |
|---|---|---|---|
| 1 | Control + cisplatin (12uM, 24h) | 39% | 35% |
| 2 | TA6496 + cisplatin (12uM, 24h) | 68% (p<0.05) | 72% (n.s.) |
| 3 | TA6440 + cisplatin (12uM, 24h) | 69% (p<0.05) | 81% (p<0.05) |
| 4 | TA6415 + cisplatin (12µM, 24h) | 68% (p<0.05) | 89% (p<0.05) |
| 5 | TA6496 (Li 10.37 mmol/kg, Mg 2.27 mmol/kg, K 16.88 mmol/kg) | 95% (n.s.) | 131% (n.s.) |
| 6 | TA6440 (Li 5.76 mmol/kg, Mg 1.26 mmol/kg, K 9.38 mmol/kg) | 96% (n.s.) | 134% (n.s.) |
| 7 | TA6415 (Li 1.73 mmol/kg, Mg 0.38 mmol/kg, K 2.81 mmol/kg) | 101% (n.s.) | 154% (n.s.) |
| 8 | K 366 mg/kg = 9.38 mmol/kg | 117% (n.s.) | 115% (n.s.) |
| 9 | Li 40 mg/kg = 5.8 mmol/kg | 97% (n.s.) | 77% (p<0.0001) |
| 10 | Mg 30.8 mg/kg = 1.27 mmol/kg | 108% (n.s.) | 98% (n.s.) |
| 11 | Li 72 mg/kg= 10.4 mmol/kg + Mg 55.4 mg/kg= 2.24 mmol/kg | 29%(p<0.0001) | 24%(p<0.0001) |
| 12 | Li 40 mg/kg = 5.8 mmol/kg + Mg 30.8 mg/kg = 1.27 mmol/kg | 24%(p<0.0001) | 29%(p<0.0001) |
| 13 | Li 40 mg/kg = 5.8 mmol/kg + K 366.7 mg/kg = 9.38 mmol/kg | 0% (p<0.0001) | 1% (p<0. 0001) |
| 14 | Li 40 mg/kg = 5.8 mmol/kg + Mg 55.4 mg/kg = 2.24 mmol/kg + K660 mg/kg = 16.88 mmol/kg | 101% (n.s.) | 94% (n.s.) |

Rows 11 to 14 of the table show that lithium combined with only one of the magnesium or potassium ions is highly toxic at 40 mg/kg, whereas it is no longer toxic in the presence of the other two ions at the same concentrations and in a variant of the algorithm on row 14. So there really is a synergistic effect of the combination of the three lithium, magnesium and potassium ions.

The ability of isolated ions to preserve the survival of sensory neurons and neurites length was tested in parallel. The results are shown in Table 3.

**Table 3.**

| | | Average percentage of sensory neuron count relative to control | Average percentage of neurite length relative to control |
|---|---|---|---|
| 1 | Control + cisplatin (12µM, 24h) | 51-57% | 63-75% |
| 2 | Control + cisplatin (12µM, 24h) + K 366 mg/kg = 9.38 mmol/kg | 58% | 91% |
| 3 | Control + cisplatin (12µM, 24h) + Li 40 mg/kg = 5.8 mmol/kg | 66% | 80% |
| 4 | Control + cisplatin (12µM, 24h) + Mg 30.8 mg/kg | 68% | 73% |

None of the individual ions significantly preserves sensory neurons from cisplatin-induced apoptosis, whereas the multi-ionic combinations tested, at similar concentrations, do. The same is true for combinations of potassium and magnesium, which do not significantly improve the survival of sensory neurons. This difference is probably due to the reduction in toxicity of multi-ionic combinations compared to isolated ions, which allows the protective effect to prevail over toxicity.

Manganese was also tested under the same conditions at concentrations ranging from 0.15 mg/kg to 1.2 mg/kg, without demonstrating any significant effect on neuron survival or neurite length.

Other combinations of lithium-magnesium-potassium according to the invention were also tested. They are shown in Table 4.

**Table 4.**

| | Magnesium | | | Lithium | | Potassium | | |
|---|---|---|---|---|---|---|---|---|
| | mg/kg | mmol/kg | Molar vs Li ratio | mg/kg | mmol/kg | mg/kg | mmol/kg | Molar vs Li ratio |
| C1 | 30.8 | 1,27 | 0.22 | 40 | 5,76 | 300 | 7, 7 | 1,33 |
| C2 | 30, 8 | 1, 27 | 0,22 | 40 | 5,76 | 500 | 12,79 | 2,22 |
| C3 | 30, 8 | 1, 27 | 0,22 | 40 | 5,76 | 366,7 | 9,38 | 1, 63 |
| C4 | 30, 8 | 1, 27 | 0,22 | 40 | 5,76 | 350 | 9 | 1, 56 |
| C5 | 5, 97 | 0, 2 | 0, 14 | 12 | 1, 7 | 110 | 2,8 | 1, 63 |
| C6 | 9, 2 | 0,37 | 0,24 | 12 | 1, 7 | 110 | 2,8 | 1,63 |
| C7 | 9, 2 | 0,37 | 0, 24 | 12 | 1, 7 | 118,65 | 3,03 | 1,76 |
| C8 | 9, 2 | 0,37 | 0, 24 | 12 | 1, 7 | 115,25 | 2,94 | 1,73 |
| C9 | 46 | 1, 9 | 0,22 | 60 | 8, 6 | 593,19 | 1, 52 | 1,76 |
| C10 | 62,73 | 2,56 | 0, 30 | 60 | 8,6 | 511,89 | 13,09 | 2, 12 |
| C11 | 67.21 | 2, 76 | 0, 32 | 60 | 8,6 | 550 | 14,07 | 1, 64 |
| C12 | 30, 8 | 1, 3 | 0,22 | 40 | 5,76 | 600 | 15,3 | 2, 66 |
| C13 | 30, 8 | 1, 3 | 0,22 | 40 | 5,76 | 250 | 6, 4 | 1, 11 |
| C14 | 50 | 2, 10 | 0, 36 | 40 | 5,76 | 366,7 | 9,38 | 1, 63 |
| C15 | 15 | 0, 6 | 0,11 | 40 | 5,76 | 366,7 | 9,38 | 1,63 |
| C16 | 50 | 2, 1 | 0, 36 | 40 | 5,76 | 250 | 6, 4 | 1, 11 |

The compositions C12 to C16 are outside the ranges of molar ratios according to the invention, while the other compositions, C1 to C11, are representative of the algorithm of the invention.

The effect of ion combinations on sensory neuron survival and neurite length was evaluated under the same conditions as described above. The solutions are all prepared from chlorides of the ions tested.

The results are shown in Table 5.

**Table 5.**

| | Average percentage of sensory neuron count relative to control |
|---|---|
| Control + cisplatin (12 µm, 24h) | 53.1 % |
| Control + cisplatin (12 µm, 24h) + C1 | 82.13%** (pp<0.01) |
| Control + cisplatin (12 µm, 24h) + C2 | 81.66%** (pp<0.01) |
| Control + cisplatin (12 µm, 24h) + C3 | 79.42%* (p<0.05) |
| Control + cisplatin (12 µm, 24h) + C4 | 78.47%* (p<0.05) |
| Control + cisplatin (12 µm, 24h) + C5 | 68.71%* (p<0.05) |
| Control + cisplatin (12 µm, 24h) + C6 | 65.05%* (p<0.05) |
| Control + cisplatin (12 µm, 24h) + C7 | 69.51%** (pp<0.01) |
| Control + cisplatin (12 µm, 24h) + C8 | 66.06%* (p<0.05) |
| Control + cisplatin (12 µm, 24h) + C9 | 90.21%** (pp<0.01) |
| Control + cisplatin (12 µm, 24h) + C10 | 84.18%** (pp<0.01) |
| Control + cisplatin (12 µm, 24h) + C11 | 80.04%* (p<0.05) |
| Control + cisplatin (12 µm, 24h) + C12 | 69.53% (n.s.) |
| Control + cisplatin (12 µm, 24h) + C13 | 69.13% (n.s.) |
| Control + cisplatin (12 µm, 24h) + C14 | 70.77% (n.s.) |
| Control + cisplatin (12 µm, 24h) + C15 | 67.69% (n.s.) |
| Control + cisplatin (12 µm, 24h) + C16 | 62.19% (n.s.) |

The above results demonstrate that the compositions of the invention significantly reduce the apoptosis of sensory neurons induced by cisplatin on all claimed beaches. When potassium and / or magnesium are below and/or above the ranges of molar ratios according to the invention (C12 to C16), the combination no longer works significantly.

### Reference Example 8 - Neuroprotective effect of a multi-ionic complex, at 3 different concentrations on primary myelinated rat sensory neurons, neurites and myelin sheaths after cisplatin injury

### Example 8a - Effect of the multi-ionic complex on the length of the myelin sheath of sensory neurons (curative protocol)

Treatment with multi-ionic complex, at 3 different concentrations TA6496, TA6440 and TA6415 for 24 hours on healthy cells does not modulate the length of the myelin sheath of sensory neurons (respectively 89%, 97% and 89% of the control).

Cisplatin at 12 µM induces a significant decrease in the length of the myelin sheath of sensory neurons (78% of myelin loss with p <0.001).

Treatment with multi-ionic complex, at 2 different concentrations TA6440 and TA6415 significantly preserves the length of the myelin sheath of sensory neurons from cisplatin lesions for 24 hours (respectively 46% with p <0.05 and 34% myelin loss with p <0.01).

However, treatment with the TA6496 multi-ionic complex induces a slight but not significant increase in myelin sheath length after cisplatin injury for 24 hours (66% of myelin loss).

### Example 8b - Neuroprotective effect of a multi-ionic complex, at 3 different concentrations on primary myelinated rat sensory neurons, neurites and myelin sheaths after cisplatin injury: protection protocol

The primary endpoint was the measurement of sensory neurons survival, the assessment of neurite length and myelin sheath length of sensory neurons after cisplatin intoxication and without intoxication.

Cis-diammineplatinum (II) dichloride (cisplatin; Sigma, ref: P4394, lot: MKCL0026) was reconstituted in medium at 10 mg/ml (stock solution). The control environment was prepared under the same conditions. After 12 days of culture with AA, primary sensory neurons were pretreated for 2 hours with a 3-concentration multi-ionic complex and then intoxicated with cisplatin. The cisplatin preparation was used on neurons at a final concentration of 12 µM diluted in control medium for 24 hours of incubation.

The concentrations of the multi-ionic complex were:
1. TA6496: Li 10.37 mmol/kg, Mg 2.27 mmol/kg, K 16.88 mmol/kg
2. TA6440: Li 5.76 mmol/kg, Mg 1.26 mmol/kg, K 9.38 mmol/kg
3. TA6415: Li 1.73 mmol/kg, Mg 0.38 mmol/kg, K 2.81 mmol/kg

The following conditions were assessed:
- Control medium
- Control + cisplatin (12uM, 24h)
- Multi-ionic complex, at 3 different concentrations TA6496, TA6440 or TA6415 + cisplatin (12uM, 24h)
- Multi-ionic complex, at 3 different concentrations TA6496, TA6440 or TA6415.

This study was performed with 6 wells per condition and duplicated for validation of results. The data included and analysed are the combined results of the 2 studies.

After 24 hours of treatment in the presence or absence of cisplatin, the cells were fixed with a solution of acetic acid / ethanol (5/95) for 5 min at -20°C, the control conditions were also fixed following the same procedure. The cells were then permeabilized and the non-specific sites were saturated with saline phosphate buffer (PBS; PanBiotech; ref: P04-36500, Batch: 2620121) containing 0.1% saponin (Sigma; ref: S7900, Batch: BCBL8667V) and 1% FCS for 15 min at room temperature. The cells were then incubated with a polyclonal anti-rabbit neurofilament antibody (NF; 1/500, Sigma; ref: N0142, Batch: 083M4833) and with an anti-MAG mouse monoclonal antibody (1/400, Sigma; ref: MAB1567, Batch: 3227322) in a PBS solution containing 1% FCS, 0.1% saponin, for 12 hours at 4°C.

These antibodies were revealed with a goat anti-mouse Alexa Fluor 488 (1/400, Molecular probe, ref: A11001, 2247988) and a goat anti-rabbit IgG Alexa Fluor 633 (1/400, Molecular probe, ref: A21070, Lot: 1700326) in PBS with 1% FCS, 0.1% saponin, for 1 hour at room temperature. The cell nuclei were labeled with a fluorescent marker (Hoechst solution, Sigma; ref: H-33258, lot: 046M4048V) in the same solution.

For each condition, 20 pictures per well were taken using InCell AnalyzerTM 2200 (GE Healthcare) with 20x magnification. The images of each crop well were taken under the same conditions. The analysis was performed using Developer software (GE Healthcare). A total of 6 data per experimental condition were provided for each of the 2 studies.

Data were expressed on average ± e.g. (based on 6 data per condition per crop for 2 crops). An overall analysis of the data was performed using a one-way analysis of variance (ANOVA) followed by the Dunnett test. The significance level is set at p <0.05.

### Effect of the multi-ionic complex on the survival of sensory neurons

Treatment with the multi-ionic complex, at 3 different concentrations TA6496, TA6440 and TA6415 for 24 hours on healthy cells does not modulate cell survival (respectively 91%, 89% and 95% of the control).

Cisplatin at 12 µM induces a significant decrease in neuron survival (64% of cell death with p <0.0001).

Pretreatment with the multi-ionic complex, at 3 different concentrations TA6496, TA6440 and TA6415, two hours before and during the 24 hours of cisplatin injury, partially and significantly saves neurons from cell death (respectively 35% with p <0.0001, 30% with p <0.0001 and 30% cell death with p <0.0001).

### Effect of the multi-ionic complex on the length of neurites of sensory neurons

Treatment with the multi-ionic complex at concentration TA6496 for 24 hours, has no impact on the length of neurites of sensory neurons (111% of the control).

Treatment with the multi-ionic complex at TA6440 concentration for 24 hours induces a slight but not significant increase in neurite length (129% of the control).

Treatment with the multi-ionic complex at concentration TA6415 for 24 hours significantly increases the length of neurites of sensory neurons (140% of control with p <0.01).

Cisplatin at 12 µM induces a significant decrease in the neurites length of sensory neurons (51% loss of neurites with p <0.0001).

A two-hour pretreatment with the multi-ionic complex, at 3 different concentrations TA6496, TA6440 and TA6415 is able to effectively preserve the length of the neurites of the sensory neurons of the cisplatin lesion for 24 hours (respectively 83% with p <0.01, 100% with p <0.0001 and 102% of the control with p <0.0001).

### Effect of the multi-ionic complex on the length of the myelin sheath of sensory neurons

Treatment with the multi-ionic complex, at 3 different concentrations TA6496 and TA6415 for 24 hours on healthy cells induce a slight but not significant increase in the length of the myelin sheath of sensory neurons (respectively, 138% and 129% of the control). Treatment with the multi-ionic complex at concentration TA6440 for 24 hours on healthy cells has no impact on the length of the myelin sheath (111% of the control).

Cisplatin at 12 µM induces a significant decrease in the length of the myelin sheath of sensory neuron extensions (69% of myelin loss with p <0.0001).

A two-hour pretreatment with the multi-ionic complex, at 3 different concentrations TA6496, TA6440 and TA6415 significantly preserves the length of the myelin sheath of sensory neurons from cisplatin lesion for 24 hours (respectively 29% with p <0.001, 37% with p <0.01 and 29% myelin loss with p <0.001).

In comparison, none of the ions isolated at the concentrations used in TA6496, TA6440 and TA6415 demonstrated a significant protective effect on myelin sheath length.

Manganese was also tested under the same conditions at concentrations ranging from 0.15 mg/kg to 1.2 mg/kg, without demonstrating any significant effect on myelin sheath length.

### Example 9 - anti-inflammatory effect in vitro on human mononuclear blood cells:

The following compositions were tested:
- TA6496 & TA64128: ionic complex (Mg 0.2/Li 1/K 1.6)
- Li alone (chloride)
- Mg alone (chloride)
- K alone (chloride)
- C17-C18 multi-ionic complex (chlorides): (Mg 0.22/Li 1/K 1.3)
- C19-C20 multi-ionic complex (chlorides): (Mg 0.14/Li 1/K 1.6)
- C21-C22 multi-ionic complex (chlorides): (Mg 0.22/Li 1/K 1.76)

Methods: Mononuclear blood cells are isolated from the blood of healthy donors through Ficoll density gradient (Ficoll-Paque PLUS; GE Healthcare; 11778538) with density gradient centrifuge tubes (SepMate; StemCell Technologies; 85450). The mononuclear blood cells are deposited (200000 cells per well) in 96-well plates, pretreated either with the vehicle (0.179% PBS) or with a composition according to the invention or ions alone (see Table 6) for 2 hours, then stimulated for 6 hours without (vehicle 0.002% H2O) or with lipopolysaccharides (LPS at 100 ng / mL; Invivogen, tlrl-b5lps) at 37°C, 5% CO2 in an RPMI medium containing 10% fetal calf serum and 1% penicillin/streptomycin. After 6 hours of incubation, the culture supernatant is collected and stored at - 20°C until cytokine analysis. The level of cytokines (IL-1β and IL-18) is quantified in the culture supernatant by ELISA according to the manufacturer's instructions (R&D Systems; DY201, DY318-05). Table 6 summarizes the percentage of IL-1β and IL-18 cytokines measured for each condition relative to the vehicle (ns: not significant)

**Table 6.**

| | [Li] mg/kg | Mg Molar vs Li Ratio | Li | K Molar vs Li ratio | Results IL-1β | Results IL-18 |
|---|---|---|---|---|---|---|
| Vehicle | | | | | 100% | 100% |
| TA6496 | 96 | 0.2 | 1 | 1.6 | 76.32%*** (p<0.001) | 69.27%** * (p<0.001 ) |
| TA64128 | 72 | 0.2 | 1 | 1.6 | 81.30 %** (p<0. 01) | 74.72%** (pp<0.01 ) |
| Mg - 55.4 mg/L | | NA | NA | NA | 100.91 % ns | 102.41 % ns |
| Mg - 73.9 mg/L | | NA | NA | NA | 97.41 % ns | 95.28 % ns |
| Li | 72 | NA | NA | NA | 101.88 % ns | 102.00 % ns |
| Li | 96 | 0 NA | NA | NA | 87.10 % ns | 116.15 % ns |
| K - 660 mg/L | | NA | NA | NA | 80.52 % ns | 72.48 % ns |
| K - 880 mg/L | | NA | NA | NA | 85.31 % ns | 69.71 % ns |
| C17 | 72 | 0.22 | 1 | 1.3 | | 70.88% * (pp<0.05 ) |
| C18 | 96 | 0.22 | 1 | 1.3 | 71.63 %* (p<0.05) | 58.88 %*** (p<0.001 ) |
| C19 | 72 | 0.14 | 1 | 1.63 | | 70.52% * (pp<0.05 ) |
| C20 | 96 | 0.14 | 1 | 1.63 | | 61.50 %** (pp<0.01 ) |
| C21 | 72 | 0.22 | 1 | 1.76 | | 60.64% ** (pp<0.01 ) |
| C22 | 96 | 0.22 | 1 | 1.76 | | 52.42 %*** (p<0.001 ) |

### Effect of TA6496 & TA64128 compounds

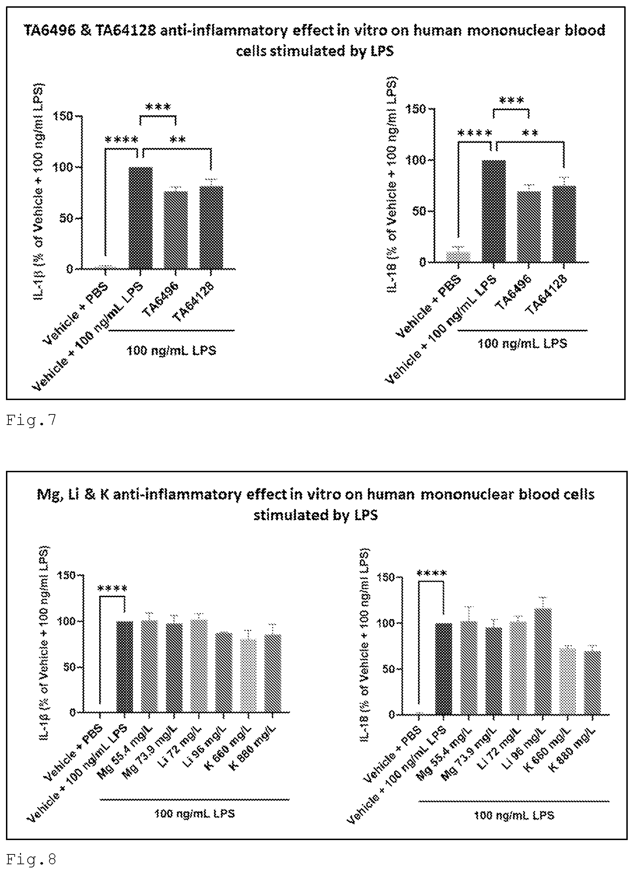

Stimulation of mononuclear blood cells by LPS (100ng/mL) induces a significant increase in IL-1β and IL-18 levels in the culture supernatant. TA6496 and TA64128 significantly reduced IL-1β and IL-18 levels in a concentration-dependent manner (Figure 7).

The compositions of the invention therefore demonstrate significant anti-inflammatory properties on human immune cells in a pro-inflammatory context.

### Effect of Mg, Li and K ions alone

Stimulation of mononuclear blood cells by LPS induced a significant increase in IL-1β and IL-18 levels in the culture supernatant. Neither magnesium, lithium, nor potassium, alone, demonstrated significant reductions in IL-1β and IL-18 levels. (Figure 8)

### Effect of C17 to C22 chloride-based multi-ionic complexes: LPS

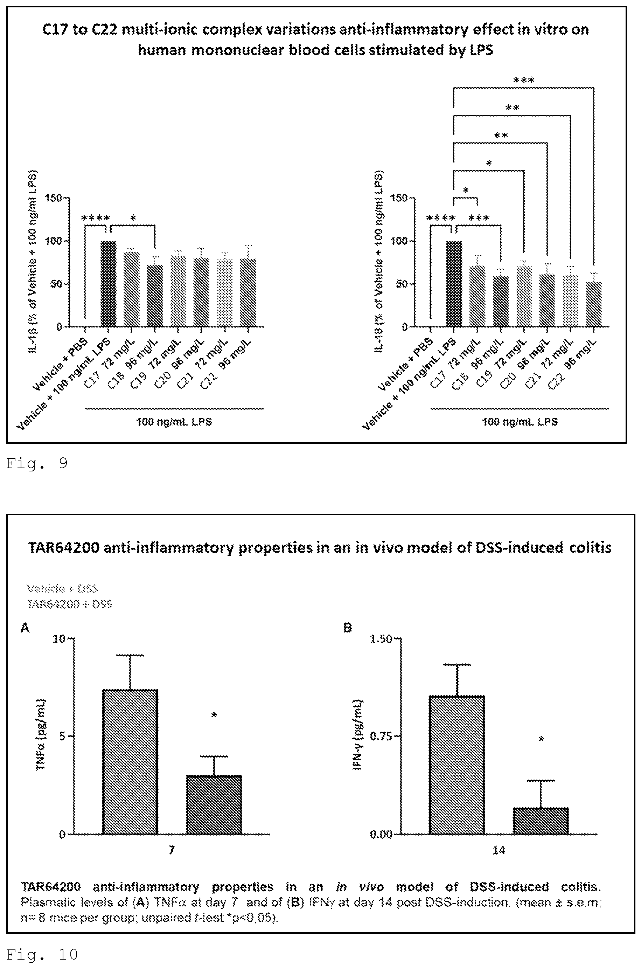

stimulation of mononuclear blood cells induced a significant increase in IL-1β and IL-18 levels in the culture supernatant. The C17 to C22 ion complexes all demonstrated significant reductions in IL-18 levels and the C18 complex at 96mg/L lithium demonstrated a significant reduction in IL-1β. The C1 to C22 ion complexes therefore demonstrate significant anti-inflammatory properties on human immune cells in a pro-inflammatory context. (Figure 9)

### Example 10 - effect of the multi-ionic complex of the invention administered orally on an in vivo model of inflammation

The Dextran Sodium Sulfate (DSS)-induced acute ulcerative colitis model was used. TNF-α and IFNγ markers were monitored.

TAR64 is the oral form of TA64.

Induction: Dextran Sulfate Sodium (DSS) is administered for 7 days, starting on day 1, followed by 5 days of washing (without DSS) to induce ulcerative colitis. The appropriate concentration of DSS (4%) is added to drinking water, available at will to animals, and is renewed every 2 days, according to standard procedures for induction of acute ulcerative colitis.

Buprenorphine (0.5mg/kg) is administered to reduce animal discomfort.

Animals: The experiment was performed on female syngeneic mice of the Balb/c breed. The mice are housed in groups of 4 according to the study groups. Each mouse is uniquely identified. The animals are housed in ventilated cages (type II (16x19x35 cm, floor area = 500cm2) under the following conditions:
- Room temperature (22±2°C).
- Hygrometry (55±10%).
- Photoperiod (12:12-hours day-night cycle 7am:7pm).
- Unlimited food and water available.

The mice were able to acclimatize to their environment for 7 days before the experiment began. If required by the procedure to be performed, mice are anesthetized by inhalation of isoflurane. The mice receive an analgesic treatment: buprenorphine (0.5mg/kg) is administered to reduce the discomfort of the animals during the induction of ulcerative colitis.

Groups and treatments: all treatments started on day 1 and were administered according to groups and Table 7 below until the end of the study (day 14).

**Table 7.**

| Group | Number of mice | DSS processing | Dose | Mode of administration |
|---|---|---|---|---|
| 1 | 8 | Yes | Vehicle | Daily PO |
| 2 | 8 | Yes | TAR64100 | Daily PO |
| 3 | 8 | Yes | TAR64200 | Daily PO |
| 4 | 8 | Yes | TAR64400 | Daily PO |

The daily doses administered by elements are listed in Table 8 below:

**Table 8.**

| Daily dose | TAR-64100 | TAR-64200 | TAR-64400 |
|---|---|---|---|
| Lithium mg/kg | 0,88 | 1,76 | 3,51 |
| Magnesium mg/kg | 0,67 | 1,35 | 2,69 |
| Potassium mg/kg | 8,05 | 16,11 | 32,21 |

On day 14, ~200µL of blood is collected from the retroorbital area of all animals, under anesthesia. Blood is transferred into EDTA-K3 tubes and gently mixed by inversion (by hand) 8-10 times. The blood is centrifuged at 800g for 5 minutes at 2-4°C. The plasma is separated, transferred into labeled polypropylene tubes and centrifuged at 10000g for 5 minutes at 2-4°C. The recovered plasma is separated, transferred to labelled polypropylene tubes and immediately frozen on dry ice or in a freezer at -80°C and stored until cytometric bead array (CBA) analysis.
CBA analysis: CBA analysis is performed using a CBA FLAX mouse kit on plasma according to the manufacturer's instructions. (TNF-α; BN; 558299) (IFNY; BD; 558296)
Results: DSS-induced ulcerative colitis induced a significant increase in plasma levels of TNF-α and IFNY on days 7 and 14. TAR64200 treatment significantly reduces TNF-α at day 7 and IFNY day 14. TAR64200 therefore demonstrates its anti-inflammatory properties in vivo in an ulcerative colitis model. (Figure 10)

### Example 11 - in vivo potassium toxicity test

Objective: In vivo toxicological study.

TAV64 is the injectable form of the compound TA64.

The TAV64 composition is composed of lithium, potassium and magnesium, all 3 elements in the form of chlorides: LiCl, KCl and MgCl₂, in a ratio 0.2-1-1.6 (like the TA64 compositions illustrated in Table 1). Regarding the toxicity of these 3 salts entering in TAV64 composition, KCl has the lowest intravenous lethal dose 50 (LD50) over 24 hours in rats, LD50 = 142mg/kg (Chemical Economics Handbook (CEH) Marketing Research Reports (1999). SRI International, p. 764.1000 I.), and is therefore potentially the most critical in intravenous administration of TAV64.

To determine whether the combination of the 3 mineral chlorides has an impact on the 24-hour LD50 of KCl, TAV64 was injected into Wistar male rats under the following conditions:
A first group of 3 male Wistar rats received a total dose of the TAV64 composition comprising 127 mg/kg of KCl as an intravenous injection over 24 hours, i.e. at 90% of the LD50 of KCl, and all rats survived.
A second group of 3 male Wistar rats received a total dose of TAV64 comprising 288 mg/kg KCl as an intravenous injection over 24 hours, i.e. 203% of the LD50 of KCl, and all rats survived.
A third group of 8 male Wistar rats was treated with lipopolysaccharide (LPS) by intraperitoneal injection to cause systemic inflammation and received a total dose of TAV64 composition comprising 240 mg / kg of KCl intravenously injected over 24 hours, 69% higher than the LD50 of KCl, and 5 out of 8 rats survived.

In conclusion, TAV64 demonstrated high tolerance and non-toxicity in vivo in the Wistar rat. The composition based on 3 chlorides of Li, Mg and K allowed to administer up to 203% of the LD50 of KCl isolated in healthy rats and to raise the LD50 of KCl injected into sick rats by 69% in a sepsis induction model, confirming that the composition of TAV64 positively impacts the LD50 of KCl and that TAV64 has a large margin of safety for its use by intravenous injection.

## Claims

1. Multi-ionic composition for use in the treatment or prevention of inflammation comprising mineral and/or organic salts, including at least one lithium, magnesium and potassium salt, **characterized by** the following molar ratios:
lithium 1 - magnesium [0.13 - 0.34] - potassium [1.20-2.40], wherein said treatment or prevention comprises oral or parenteral administration of said multi-ionic composition.

2. A composition for use according to claim 1, wherein lithium is present at a concentration between 0.01 and 100 mmol / kg.

3. A composition for use according to one of claims 1 and 2, wherein the lithium salt is a mineral salt, for example lithium chloride, lithium hydroxide or lithium carbonate.

4. A composition for use according to one of claims 1 and 2, wherein the lithium salt is an organic salt, for example lithium citrate, lithium gluconate or lithium orotate.

5. A composition for use according to one of claims 1 to 4, wherein the magnesium salt is a mineral salt, for example a chloride, a hydroxide, a sulfate, an oxide, a silicate or a carbonate.

6. A composition for use according to one of claims 1 to 4, wherein the magnesium salt is an organic salt, for example magnesium bisglycinate, magnesium malate, magnesium glycerophosphate, magnesium stearate, magnesium ascorbate, magnesium taurate, magnesium citrate, magnesium gluconate or magnesium taurinate.

7. A composition for use according to one of claims 1 to 6, wherein the potassium salt is a mineral salt, for example potassium chloride, potassium bromide, potassium iodide, potassium phosphate, potassium carbonate, potassium hydroxide, potassium silicate.

8. A composition for use according to one of claims 1 to 6, wherein the potassium salt is an organic salt, for example potassium gluconate, potassium citrate, potassium malate, potassium glycerophosphate, potassium lactate, potassium pidolate, potassium aspartate or potassium gluconate.

9. Composition for use according to one of claims 1 to 8 for the prevention and / or treatment of chronic inflammation or acute inflammation.

10. A composition for use according to claim 9, for use in a method of treating or preventing a condition selected from polyarthritis, osteoarthritis, atherosclerosis, ulcerative colitis, Crohn's disease or irritable bowel syndrome.

11. Composition for use according to claim 9 for use in a method of treating or preventing a condition selected from chronic neuroinflammation.

12. A composition for use according to claim 9 or 11 for use in a method of treating or preventing a condition selected from multiple sclerosis, amyotrophic lateral sclerosis, Alzheimer's disease, Huntington's disease or migraine.

13. A composition for use according to claim 9, for use in a method of treating or preventing a condition selected from stroke, infarction, sepsis, burns, systemic inflammatory response syndrome, Covid, trauma or postoperative inflammation.

14. A composition for use according to one of claims 1 to 13 ,
(i)further comprising at least one additive selected from stabilizers, emulsifiers, preservatives, antioxidant and/or gelling agents,
and/or
(ii) further comprising one or more other mineral or organic salt, such as a salt of zinc, manganese, copper and/or silicon.

## Patentansprüche

1. Multi-ionische Zusammensetzung zur Anwendung bei der Behandlung oder Vorbeugung von Entzündungen, die mineralische und/oder organische Salze enthält, darunter mindestens ein Lithium-, Magnesium- und Kaliumsalz, **gekennzeichnet durch** folgende Molarverhältnisse:
Lithium 1 - Magnesium [0,13 - 0,34] - Kalium [1,20-2,40], wobei die Behandlung oder Prävention die orale oder parenterale Verabreichung der genannten multiionischen Zusammensetzung umfasst.

2. Eine Zusammensetzung für die Verwendung gemäß Anspruch 1, bei der Lithium in einer Konzentration zwischen 0,01 und 100 mmol/kg vorhanden ist.

3. Eine Zusammensetzung für die Verwendung gemäß einer der Ansprüche 1 und 2, bei der das Lithiumsalz ein Mineralsalz ist, zum Beispiel Lithiumchlorid, Lithiumhydroxid oder Lithiumcarbonat.

4. Eine Zusammensetzung zur Verwendung gemäß einer der Ansprüche 1 und 2, bei der das Lithiumsalz ein organisches Salz ist, zum Beispiel Lithiumcitrat, Lithiumglukonat oder Lithiumorotat.

5. Eine Zusammensetzung zur Verwendung gemäß einer der Ansprüche 1 bis 4, wobei das Magnesiumsalz ein Mineralsalz ist, zum Beispiel ein Chlorid, ein Hydroxid, ein Sulfat, ein Oxid, ein Silikat oder ein Karbonat.

6. Eine Zusammensetzung zur Verwendung gemäß einer der Behauptungen 1 bis 4, bei der das Magnesiumsalz ein organisches Salz ist, zum Beispiel Magnesiumbisglycinat, Magnesium-Malat, Magnesiumglycerophosphat, Magnesiumstearat, Magnesiumascorbat, Magnesium-Taurat, Magnesiumcitrat, Magnesiumglukonat oder Magnesium-Taurinat.

7. Eine Zusammensetzung zur Verwendung gemäß einem der Ansprüche 1 bis 6, bei der das Kaliumsalz ein Mineralsalz ist, zum Beispiel Kaliumchlorid, Kaliumbromid, Kaliumiodid, Kaliumphosphat, Kaliumcarbonat, Kaliumhydroxid, Kaliumsilikat.

8. Eine Zusammensetzung zur Verwendung gemäß einem der Ansprüche 1 bis 6, bei der das Kaliumsalz ein organisches Salz ist, zum Beispiel Kaliumglukonat, Kaliumcitrat, Kalium-Malat, Kaliumglycerophosphat, Kaliumlaktat, Kaliumpidolat, Kaliumaspartat oder Kaliumglukonat.

9. Zusammensetzung zur Verwendung gemäß einer der Ansprüche 1 bis 8 zur Vorbeugung und/oder Behandlung chronischer oder akuter Entzündung.

10. Eine Zusammensetzung für die Anwendung gemäß Anspruch 9, zur Anwendung in einer Methode zur Behandlung oder Vorbeugung einer Erkrankung, die aus Polyarthritis, Arthrose, Arteriosklerose, Colitis ulcerosa, Morbus Crohn oder Reizdarmsyndrom ausgewählt wird.

11. Zusammensetzung zur Verwendung gemäß Behauptung 9 zur Anwendung in einer Methode zur Behandlung oder Vorbeugung einer aus chronischer Neuroentzündung ausgewählten Erkrankung.

12. Eine Zusammensetzung zur Anwendung gemäß Behauptung 9 oder 11 zur Anwendung in einer Methode zur Behandlung oder Prävention einer Erkrankung, die aus Multipler Sklerose, amyotropher Lateralsklerose, Alzheimer, Huntington-Krankheit oder Migräne ausgewählt wird.

13. Eine Zusammensetzung für die Verwendung gemäß Anspruch 9, zur Anwendung in einer Methode zur Behandlung oder Prävention einer Erkrankung, die aus Schlaganfall, Infarkt, Sepsis, Verbrennungen, systemischem Entzündungsreaktionssyndrom, Covid, Trauma oder postoperativer Entzündung ausgewählt wird.

14. Eine Komposition zur Verwendung gemäß einem der Ansprüche 1 bis 13,die
(i) außerdem mindestens ein Zusatzstoff umfasst, der aus Stabilisatoren, Emulgatoren, Konservierungsstoffen, Antioxidativen und/oder Gelierungsmitteln ausgewählt wird,
und/oder
(ii) außerdem ein oder mehrere andere mineralische oder organische Salze enthalten, wie z. B. ein Salz aus Zink, Mangan, Kupfer und/oder Silizium.

## Revendications

1. Composition multi-ionique destinée au traitement ou à la prévention de l'inflammation comprenant des sels minéraux et/ou organiques, y compris au moins un sel de lithium, magnésium et potassium, **caractérisés par** les rapports molaires suivants :
lithium 1 - magnésium [0,13 - 0,34] - potassium [1,20-2,40], dans lequel ce traitement ou prévention comprend l'administration orale ou parentérale de cette composition multi-ionique.

2. Une composition à utiliser selon la revendication 1, où le lithium est présent à une concentration comprise entre 0,01 et 100 mmol / kg.

3. Une composition à utiliser selon l'une des revendications 1 et 2, où le sel de lithium est un sel minéral, par exemple chlorure de lithium, hydroxyde de lithium ou carbonate de lithium.

4. Une composition à utiliser selon l'une des revendications 1 et 2, où le sel de lithium est un sel organique, par exemple citrate de lithium, gluconate de lithium ou orotate de lithium.

5. Une composition à utiliser selon l'une des revendications 1 à 4, où le sel de magnésium est un sel minéral, par exemple un chlorure, un hydroxyde, un sulfate, un oxyde, un silicate ou un carbonate.

6. Une composition à utiliser selon l'une des revendications 1 à 4, où le sel de magnésium est un sel organique, par exemple bisglycinate de magnésium, malate de magnésium, glycérophosphate de magnésium, stéarate de magnésium, ascorbate de magnésium, taurate de magnésium, citrate de magnésium, gluconate de magnésium ou taurinate de magnésium.

7. Une composition à utiliser selon l'une des revendications 1 à 6, où le sel de potassium est un sel minéral, par exemple chlorure de potassium, bromure de potassium, iodure de potassium, phosphate de potassium, carbonate de potassium, hydroxyde de potassium, silicate de potassium.

8. Une composition à utiliser selon l'une des revendications 1 à 6, où le sel de potassium est un sel organique, par exemple gluconate de potassium, citrate de potassium, malate de potassium, glycérophosphate de potassium, lactate de potassium, pidolate de potassium, aspartate de potassium ou gluconate de potassium.

9. Composition pour une utilisation selon l'une des revendications 1 à 8 pour la prévention et/ou le traitement de l'inflammation chronique ou de l'inflammation aiguë.

10. Une composition à utiliser selon la revendication 9, pour une méthode de traitement ou de prévention d'une affection sélectionnée parmi la polyarthrite, l'arthrose, l'athérosclérose, la colite ulcéreuse, la maladie de Crohn ou le syndrome du côlon irritable.

11. Composition pour une utilisation selon la revendication 9 pour une méthode de traitement ou de prévention d'une affection sélectionnée à partir de la neuroinflammation chronique.

12. Une composition à utiliser selon la revendication 9 ou 11 pour une méthode de traitement ou de prévention d'une affection sélectionnée comme la sclérose en plaques, la sclérose latérale amyotrophique, la maladie d'Alzheimer, la maladie de Huntington ou la migraine.

13. Une composition à utiliser selon la revendication 9, pour une méthode de traitement ou de prévention d'une affection issue de l'AVC, de l'infarctus, de la septicémie, des brûlures, du syndrome de réponse inflammatoire systémique, du Covid, d'un traumatisme ou d'une inflammation postopératoire.

14. Une composition à utiliser selon l'une des revendications 1 à 13 ,
(i) comprenant en outre au moins un additif sélectionné parmi des stabilisateurs, émulsifiants, conservateurs, antioxydants et/ou agents gélifiants,
et/ou
(ii) comprenant en outre un ou plusieurs autres sels minéraux ou organiques, tels qu'un sel de zinc, de manganèse, de cuivre et/ou de silicium.
